# EUROPEAN PATENT SPECIFICATION

(11) **EP 2 451 338 B1**
(45) Date of publication and mention of the grant of the patent: **26.02.2020**
(21) Application number: 10797903.1
(22) Date of filing: 09.07.2010
(51) Int. Cl.: A61B 1/05, A61B 1/04, A61B 1/045, A61B 1/06, A61B 1/313

(54) **HAND-HELD MINIMALLY DIMENSIONED DIAGNOSTIC DEVICE HAVING INTEGRATED DISTAL END VISUALIZATION**
TRAGBARE, MÖGLICHST GERING BEMESSENE DIAGNOSEVORRICHTUNG MIT INTEGRIERTER DISTALENDE-VISUALISIERUNG
DISPOSITIF PORTATIF DE DIAGNOSTIC D ENCOMBREMENT MINIMAL AVEC VISUALISATION INTÉGRÉE À L EXTRÉMITÉ DISTALE

(30) Priority: 10.07.2009 US 501336
(43) Date of publication of application: 16.05.2012
(73) Proprietor: Trice Medical, Inc., Malvern, PA 19355 (US)
(72) Inventor: SCHULTZ, Eric E., Menlo Park California 94025 (US); CYBULSKI, James S., Menlo Park California 94025 (US); OUYANG, Xiaolong, Palo Alto California 94303 (US)
(74) Representative: Invent Horizon IP
(86) International application number: PCT/US2010/041502
(87) International publication number: WO 2011/006052

(56) References cited:
- EP-A2- 1 252 859
- WO-A1-00/09001
- US-A- 5 873 814
- US-A- 5 928 137
- US-A1- 2002 080 248
- US-A1- 2005 096 504
- US-A1- 2006 004 258
- US-A1- 2007 129 604
- US-A1- 2007 276 183
- US-A1- 2008 062 429
- US-A1- 2008 091 064
- US-A1- 2008 207 996
- US-B1- 6 234 955
- US-B1- 6 315 712

## Description

### INTRODUCTION

For the practitioner, the field of diagnostic imaging, for example endoscopy, has allowed for the viewing of objects, internal mechanisms and the like with minimal disruption to the subjects necessarily penetrated to view the afore mentioned objects and mechanisms. Such imaging tools have been used in a wide variety of settings for detailed inspection, including but not limited to the use and application in the field of medicine.

Of particular challenge in the case of using imaging, for example, in the medical field, is the vast amount of equipment typically required, the maintenance of such equipment, and the cabling required for connection to other systems. Among the vast array of equipment required to accomplish an imaging application found in the prior art includes monitor systems, lighting systems and power systems. In addition these systems may be permanently or semi-permanently installed in small offices or operation rooms, for example, which require said offices and rooms to be adapted in potentially a less than ideal fashion so as to accommodate the cumbersomeness of the imaging equipment. In addition, this challenge of the needed installation of imaging systems components may require the duplication of such imaging systems in other offices and rooms as required.

Compounding the above mentioned problem is the requirement that many of these imaging system components must utilize a cabling means to function. These cables that transfer electrical, optical and mechanical means, for example, may physically interfere with objects and persons in the room such as a patient. In some cases, cables for light transmission, for example fiber optic cables, that are rather inflexible may break if over-flexed and thus compromise the outcome of the imaging application.

An additional challenge for imaging technology found in the prior art is the use of external monitoring of the imaging that may be located some distance from the practitioner. As is the case, the practitioner would then be required to view the monitoring of the imaging application in one direction while physically introducing or utilizing the imaging means in a different direction, thus potentially compromising the detail and accuracy of the use of the imaging tool.

Another problem with such imaging systems is that they may require external power. This power must be located relatively proximate to the location of the power outlets and the required voltage available. Since various countries do not share a common power adapter means, or the same voltage output, additional adapters must be utilized for functionality of these systems.

Another challenge faced by imaging systems is satisfaction of the goals of sterility and reuseability. Imaging systems must be sterile in order to be employed for their intended applications. While sterility can be accomplished by using a device only once, such approaches are wasteful. However, reusing a device poses significant challenges with repect to maintaining sterility.

Documents US 2002/080248 and US 2005/096504 represent the closest prior art.

### SUMMARY

Hand-held minimally dimensioned diagnostic devices having integrated distal end visualization are provided. The invention of the present application relates to an internal tissue visualization device comprising: a hand-held control unit comprising a hand piece, the hand-held control unit comprising a luer connector configured to receive a fluid;an integrated visualization sensor configured to collect images of an internal tissue site and transmit the images to a monitor;an elongated member comprising a lumen wherein the lumen is configured to deliver the fluid to a tissue site and wherein the elongated member comprises a proximal end operatively coupled to the hand-held control unit and a minimally dimensioned distal end having integrated the visualization sensor; and the distal end of the elongated member further comprising an integrated illuminator arranged concentrically around the visualization sensor, wherein the integrated illuminator has a crescent configuration.

### BRIEF DESCRIPTION OF THE FIGURES

FIG. 1A is a side view of one embodiment of a portable diagnostic tool.
FIG. 1B is a section view of the portable diagnostic tool of FIG. 1A.
FIG. 1C is a perspective view of the portable diagnostic tool of FIG. 1A.
FIG. 1D is an exploded view of the portable diagnostic tool of FIG. 1A .
FIG. 1E is a perspective, exploded view of the portable diagnostic tool of FIG. 1A
FIG. 1F is a close-up, side view of the portable diagnostic tool of FIG. 1A showing a port for introducing material, medicine and implant.
FIG. 1G is a perspective view of the portable diagnostic tool of FIG. 1A, with the top of the device housing removed to show the geared mechanism between a motor and the elongated member for the purpose of rotating the elongated member along its axis relative to the hand-held control unit, and connections for monitor, lighting, camera and motor to a control board, within the distal portion of the hand piece.
FIG. 1H is one embodiment of the elongated member to motor junction of the portable diagnostic tool of FIG. 1G that shows a friction-based drive connection between a motor and the elongated member for the purpose of rotating the elongated member along its axis relative to the hand-held control unit.
FIG. 1I is a perspective view of the control board, electronics, connections, buttons and switching controls of the portable diagnostic tool of FIG. 1D.
FIG. 1J is a side view of the portable diagnostic tool of FIG. 1A that shows a disconnected elongated member portion of the device from the hand-held control unit.
FIG. 1K is a side view of the portable diagnostic tool of FIG. 1A that shows a disconnected catheter portion of the device and a disconnected monitor portion of the device from the hand-held control unit.
FIG. 2A is a section view of the distal tip of the elongated member of the portable diagnostic tool of FIG. 1A that shows camera, lighting, prism lens and electrical connection.
FIG. 2B shows an embodiment of an image filter within the distal tip of the catheter of FIG. 2A.
FIG. 2C shows another embodiment of an image filter within the distal tip of the elongated member of FIG. 2A.
FIG. 2D is a section view of the distal tip of the elongated member of the portable diagnostic tool of FIG. 1A that shows camera, lighting, flat cover lens and electrical connection.
FIG. 2E shows an image filter configuration according to one embodiment within the distal tip of the catheter of FIG. 2D.
FIG. 2F shows another image filter configuration according to one embodiment within the distal tip of the catheter of FIG. 2D.
FIG. 3A is a front view of the distal tip of an elongated member of the portable diagnostic tool of FIG. 1A that shows an eccentric arrangement between a camera and an integrated illuminator.
FIG. 3B is a front view of the distal tip of the elongated member of the portable diagnostic tool of FIG. 1A that shows an eccentric arrangement between a camera and integrated illuminator, with an additional arrangement of sensors or ports.
FIG. 3C is a front view of the distal tip of an elongated member of a portable diagnostic tool of the invention that shows a concentric arrangement between a camera and an integrated illuminator.
FIG. 3D is a front view of the distal tip of an elongated member of a portable diagnostic tool of the invention that shows a concentric arrangement between a camera and an integrated illuminator, with an additional arrangement of sensors or ports.
FIG. 3E is a section view of the top view of the portable diagnostic tool of FIG. 1A that shows a wiring diagram for a sensor located at the distal tip of the elongated member and connecting to the control board, according to one embodiment of the invention.
FIG. 3F is a section view of the top view of the portable diagnostic tool of FIG. 1A that shows a conduit diagram for a port located at the distal tip of the elongated member and connecting to the port of FIG. 1F, according to one embodiment.
FIG. 4A is a side view of an embodiment for a sterile sheath for the portable diagnostic tool of FIG. 1A that shows an integral monitor cover, control cover, connection to a detachable elongated member, and sealable opening.
FIG. 4B is a side view of an embodiment for a sterile sheath for the portable diagnostic tool of FIG. 1A that shows an integral control cover, connection to a detachable elongated member, and sealable opening.
FIG. 4C is a side view of the sterile sheath of FIG. 4A surrounding the portable diagnostic tool with detached elongated member of FIG. 1I that shows the integral monitor cover over the monitor of FIG. 1I, and an integral control cover over the controls of FIG. 11.
FIG. 4D is a side view of the sterile sheath of FIG. 4A conforming to the shape of the portable diagnostic tool of FIG. 1A and the opening of FIG. 4A is sealed.
FIG. 4E is a side view of the sterile sheath of FIG. 4B conforming to the shape of the portable diagnostic tool of FIG. 1J with the monitor removed but with the catheter piece attached as in FIG. 1A, and the opening of FIG. 4B is sealed.
FIG. 4F is a side view of the sterile sheath of FIG. 4B conforming to the shape of the portable diagnostic tool of FIG. 1J with the monitor removed and the monitor mount that is located on the hand piece removed but with the elongated member attached as in FIG. 1A, and the opening of FIG. 4B is sealed.
FIG. 5A shows a view of one embodiment for a flexible elongated member section in a straight orientation relative to the axis of the elongated member of FIG. 1A with a control cable.
FIG. 5B shows a view of one embodiment for a flexible elongated member section in a bent or flexed orientation relative to the axis of the elongated member of FIG. 1A with a control cable.
FIG. 5C shows a view of one embodiment for an elongated member in a bent orientation relative to the axis of the elongated member of FIG. 1A.
FIG. 6A is a section view of the distal tip of the elongated member of FIG. 2D showing low-profile biopsy tool that includes an annular member concentrically located at the distal end of the elongated member, and a cable means for actuating the annular member, according to one embodiment.
FIG. 6B is a side view of the distal tip of the elongated member of FIG. 2D showing low-profile biopsy tool that includes an annular member concentrically located at the distal end of the elongated member, and a cable for actuating the former.
FIG. 7 is a section view of the distal tip of the catheter of FIG. 2D showing a low profile cutter concentrically located to the tip of the elongated member.
FIG. 8 is a perspective view of the distal tip of the catheter of FIG. 3F illustrating one embodiment for a slidably present sensor that is in a working channel within the elongated member and can be deployed and remain in a tissue site after the portable diagnostic device of FIG 1A is removed.
FIG. 9 is a block diagram showing an embodiment of an electronic control schema for the portable diagnostic device of FIG 1A.
FIG. 10 is a block functional diagram of a stereoscopic imaging module according to one embodiment of the invention.
FIGS. 11A and 11B illustrate off-set views of that may be obtained with a single visualization sensor (FIG. 11A) or two visualization sensors (FIG. 11B).

### DETAILED DESCRIPTION

Before the present invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

Any recited method can be carried out in the order of events recited or in any other order which is logically possible.

In further describing various aspects of the invention, aspects of embodiments of the subject tissue visualization devices and systems are described first in greater detail. Next, embodiments of methods of visualizing an internal target tissue of a subject in which the subject tissue visualization systems may find use are reviewed in greater detail.

### TISSUE VISUALIZATION DEVICES AND SYSTEMS

As summarized above, aspects of the invention include internal tissue visualization systems. The internal tissue visualization systems are visualization systems that are configured to visualize an internal tissue site of a subject. As such, the systems are structured or designed to provide images of a tissue site inside of a body, such as a living body, to a user. As such, aspects of systems of the invention include internal tissue visualization devices that are useful for visualizing an internal target tissue site, e.g., a spinal location that is near or inside of an intervertebral disc (IVD). The internal tissue visualization devices of embodiments of systems of the invention are dimensioned such that at least the distal end of the devices can pass through a minimally invasive body opening. As such, at least the distal end of the devices of these embodiments may be introduced to an internal target site of a patient, e.g., a spinal location that is near or inside of an intervertebral disc, through a minimal incision, e.g., one that is less than the size of an incision employed for an access device having a outer diameter of 20 mm or smaller, e.g., less than 75% the size of such an incision, such as less than 50% of the size of such an incision, or smaller.

As summarized above, internal tissue visualization devices of the systems of the invention include an elongated member and a hand-held control unit (such as a probe piece and hand piece as described further below). With respect to the elongated member, this component of the devices has a length that is 1.5 times or longer than its width, such as 2 times or longer than its width, including 5 or even 10 times or longer than its width, e.g., 20 times longer than its width, 30 times longer than its width, or longer. The length of the elongated member may vary, and in some instances ranges from 5 cm to 20 cm, such as 7.5 cm to 15 cm and including 10 to 12 cm. The elongated member may have the same outer cross-sectional dimensions (e.g., diameter) along its entire length. Alternatively, the cross-sectional diameter may vary along the length of the elongated member.

In some instances, at least the distal end region of the elongated member of the devices is dimensioned to pass through a Cambin's triangle. By distal end region is meant a length of the elongated member starting at the distal end of 1 cm or longer, such as 3 cm or longer, including 5 cm or longer, where the elongated member may have the same outer diameter along its entire length. The Cambin's triangle (also known in the art as the Pambin's triangle) is an anatomical spinal structure bounded by an exiting nerve root and a traversing nerve root and a disc. The exiting root is the root that leaves the spinal canal just cephalad (above) the disc, and the traversing root is the root that leaves the spinal canal just caudad (below) the disc. Where the distal end of the elongated member is dimensioned to pass through a Cambin's triangle, at least the distal end of the device has a longest cross-sectional dimension that is 10 mm or less, such as 8 mm or less and including 7 mm or less. In some instances, the devices include an elongated member that has an outer diameter at least in its distal end region that is 5.0 mm or less, such as 4.0 mm or less, including 3.0 mm or less.

The elongated members of the subject tissue visualization devices have a proximal end and a distal end. The term "proximal end", as used herein, refers to the end of the elongated member that is nearer the user (such as a physician operating the device in a tissue modification procedure), and the term "distal end", as used herein, refers to the end of the elongated member that is nearer the internal target tissue of the subject during use. The proximal end is also the end that is operatively coupled to the hand-held control unit of the device (described in greater detail below). The elongated member is, in some instances, a structure of sufficient rigidity to allow the distal end to be pushed through tissue when sufficient force is applied to the proximal end of the elongate member. As such, in these embodiments the elongated member is not pliant or flexible, at least not to any significant extent.

As summarized above, the visualization devices include a visualization sensor integrated at the distal end of the elongated member, such that the visualization sensor is integrated with the elongated member. As the visualization sensor is integrated with the elongated member, it cannot be removed from the remainder of the elongated member without significantly compromising the structure and functionality of the elongated member. Accordingly, the devices of the present invention are distinguished from devices which include a "working channel" through which a separate autonomous device is passed through. In contrast to such devices, since the visualization sensor of the present device is integrated with the elongated member, it is not a separate device from the elongated member that is merely present in a working channel of the elongated member and which can be removed from the working channel of such an elongated member without structurally compromising the elongated member in any way. The visualization sensor may be integrated with the elongated member by a variety of different configurations. Integrated configurations include configurations where the visualization sensor is fixed relative to the distal end of the elongated member, as well as configurations where the visualization sensor is movable to some extent relative to the distal end of the elongated member. Movement of the visualization sensor may also be provided relative to the distal end of the elongated member, but then fixed with respect to another component present at the distal end, such as a distal end integrated illuminator. Specific configurations of interest are further described below in connection with the figures.

Visualization sensors of interest include miniature imaging sensors that have a cross-sectional area which is sufficiently small for its intended use and yet retains a sufficiently high matrix resolution. Imaging sensors of interest are those that include a photosensitive component, e.g., array of photosensitive elements that convert light into electrons, coupled to a circuitry component, such as an integrated circuit. The integrated circuit may be configured to obtain and integrate the signals from the photosensitive array and output image data, which image data may in turn be conveyed to an extra-corporeal device configured to receive the data and display it to a user. The image sensors of these embodiments may be viewed as integrated circuit image sensors. The integrated circuit component of these sensors may include a variety of different types of fucntionalities, including but not limited to: image signal processing, memory, and data transmission circuitry to transmit data from the visualization sensor to an extra-corporeal location, etc. The miniature imaging sensors may be present in a module which further includes one or more of a housing, a lens component made up of one or more lenses positioned relative to the photosensitive component so as to focus images on the photosensitive component, one or more filters, polarized members, etc. Specific types of miniature imaging sensors of interest include complementary metal-oxide-semiconductor (CMOS) sensors and charge-coupled device (CCD) sensors. The sensors may have any convenient configuration, including circular, square, rectangular, etc. Visualization sensors of interest may have a longest cross-sectional dimension that varies depending on the particular embodiment, where in some instances the longest cross sectional dimension (e.g., diameter) is 4.0 mm or less, such as 3.5 mm or less, including 3.0 mm or less, such as 2.5 mm or less, including 2.0 mm or less, including 1.5 mm or less, including 1.0 mm or less. Within a given imaging module, the sensor component may be located some distances from the lens or lenses of the module, where this distance may vary, such as 10 mm or less, including 7 mm or less, e.g., 6 mm or less.

Imaging sensors of interest may be either frontside or backside illumination sensors, and have sufficiently small dimensions while maintaining sufficient functionality to be integrated at the distal end of the elongated members of the devices of the invention. Aspects of these sensors are further described in one or more the following U.S. Patents: 7,388,242; 7,368,772; 7,355,228; 7,345,330; 7,344,910; 7,268,335; 7,209,601; 7,196,314; 7,193,198; 7,161,130; and 7,154,137.

As summarized above, the visualization sensor is located at the distal end of the elongated member, such that the visualization sensor is a distal end visualization sensor. In these instances, the visualization sensor is located at or near the distal end of the elongated member. Accordingly, it is positioned at 3 mm or closer to the distal end, such as at 2 mm or closer to the distal end, including at 1 mm or closer to the distal end. In some instances, the visualization sensor is located at the distal end of the elongated member. The visualization sensor may provide for front viewing and/or side-viewing, as desired. Accordingly, the visualization sensor may be configured to provide image data as seen in the forward direction from the distal end of the elongated member. Alternatively, the visualization sensor may be configured to provide image data as seen from the side of the elongate member. In yet other embodiments, a visualization sensor may be configured to provide image data from both the front and the side, e.g., where the image sensor faces at an angle that is less than 90° relative to the longitudinal axis of the elongated member.

Components of the visualization sensor, e.g., the integrated circuit, one or more lenses, etc., may be present in a housing. The housing may have any convenient configuration, where the particular configuration may be chosen based on location of the sensor, direction of view of the sensor, etc. The housing may be fabricated from any convenient material. In some instances, non-conductive materials, e.g., polymeric materials, are employed.

Visualization sensors may further include functionality for conveying image data to an extra-corporeal device, such as an image display device, of a system. In some instances, a wired connection, e.g., in the form of a signal cable (or other type of signal conveyance element), may be present to connect the visualization sensor at the distal end to a device at the proximal end of the elongate member, e.g., in the form of one or more wires running along the length of the elongate member from the distal to the proximal end. In some instances, the visualization sensor is coupled to a conductive member (e.g., cable or analogous structure) that conductively connects the visualization sensor to a proximal end location of the elongated member. Alternatively, wireless communication protocols may be employed, e.g., where the visualization sensor is operatively coupled to a wireless data transmitter, which may be positioned at the distal end of the elongated member (including integrated into the visualization sensor, at some position along the elongated member or at the proximal end of the device, e.g., at a location of the proximal end of the elongated member or associated with the handle of the device).

The devices include one or more illumination elements configured to illuminate a target tissue location so that the location can be visualized with a visualization sensor, e.g., as described above. A variety of different types of light sources may be employed as illumination elements (also referred to herein as illuminators), so long as their dimensions are such that they can be positioned at the distal end of the elongated member. The light sources may be integrated with a given component (e.g., elongated member) such that they are configured relative to the component such that the light source element cannot be removed from the remainder of the component without significantly compromising the structure of the component. As such, the integrated illuminators of these embodiments are not readily removable from the remainder of the component, such that the illuminator and remainder of the component form an inter-related whole. The light sources may be light emitting diodes (LEDs) configured to emit light of the desired wavelength range, or optical conveyance elements, e.g., optical fibers, configured to convey light of the desired wavelength range from a location other than the distal end of the elongate member, e.g., a location at the proximal end of the elongate member, to the distal end of the elongate member. The physical location of the light source, e.g., LED, may vary, such as any location in the elongated member, in the hand-held control unit, etc.

As with the image sensors, the light sources may include a conductive element, e.g., wire, or an optical fiber, which runs the length of the elongate member to provide for power and control of the light sources from a location outside the body, e.g., an extracorporeal control device.

Where desired, the light sources may include a diffusion element to provide for uniform illumination of the target tissue site. Any convenient diffusion element may be employed, including but not limited to a translucent cover or layer (fabricated from any convenient translucent material) through which light from the light source passes and is thus diffused. In those embodiments of the invention where the system includes two or more illumination elements, the illumination elements may emit light of the same wavelength or they may be spectrally distinct light sources, where by "spectrally distinct" is meant that the light sources emit light at wavelengths that do not substantially overlap, such as white light and infra-red light. In certain embodiments, an illumination configuration as described in copending United States Application Serial Nos. 12/269,770 and 12/269772.

Distal end integrated illuminators may have any convenient configuration. Configurations of interest have various cross-sectional shapes, including but not limited to circular, ovoid, rectangular (including square), irregular, etc. In some instances the configuration of the integrated illuminator is configured to conform with the configuration of the integrated visualization sensor such that the cross-sectional area of the two components is maximized within the overall minimal cross-sectional area available at the distal end of the elongated member. For example, the configurations of the integrated visualization sensor and illuminators may be such that the integrated visualization sensor may occupy a first portion of the available cross-sectional area of the distal end of the elongated member (such as 40% or more, including 50% or 60% or more of the total available cross-sectional area of the distal end of the elongated member) and the integrated illuminator may occupy a substantial portion of the remainder of the cross-sectional area, such as 60% or more, 70% or more, or 80% or more of the remainder of the cross-sectional area.

The integrated illuminator of the invention is arranged concentrically around the visualization sensor and has a crescent configuration. The crescent configuration may have dimensions configured to confirm with walls of the elongated member and a circular visualization sensor. In another configuration of interest, the integrated illuminator has an annular configuration, e.g., where conforms to the inner walls of the elongated member or makes up the walls of the elongated member, e.g., as described in greater detail below. This configuration may be of interest where the visualization sensor is positioned at the center of the distal end of the elongated member.

In some instances, the elongated member comprises an annular wall configured to conduct light to the elongated member distal end from a proximal end source. The distal end of this annular wall may be viewed as an integrated illuminator, as described above. In these instances, the walls of the elongated structure which collective make up the annular wall are fabricated from a translucent material which conducts light from a source apart from the distal end, e.g., from the proximal end, to the distal end. Where desired, a reflective coating may be provided on the outside of the translucent elongated member to internally reflect light provided from a remote source, e.g., such as an LED at the proximal end, to the distal end of the device. Any convenient reflective coating material may be employed.

Also of interest are integrated illuminators that include a fluid filled structure that is configured to conduct light to the elongated member distal end from a proximal end source. Such a structure may be a lumen that extends along a length of the elongated structure from a proximal end light source to the distal end of the elongated structure. When present, such lumens may have a longest cross section that varies, ranging in some instances from 0.5 to 4.0 mm, such as 0.5 to 3.5 mm, including 0.5 to 3.0 mm. The lumens may have any convenient cross-sectional shape, including but not limited to circular, square, rectangular, triangular, semi-circular, trapezoidal, irregular, etc., as desired. The fluid filled structure may be filled with any convenient translucent fluid, where fluids of interest include aqueous fluids, e.g., water, saline, etc., organic fluids, such as heavy mineral oil (e.g., mineral oil having a specific gravity greater than or equal to about 0.86 and preferably between about 0.86 and 0.905), and the like.

As indicated above, certain instances of the integrated illuminators are made up of an elongated member integrated light conveyance structure, e.g., optical fiber, light conductive annular wall, light conducting fluid filled structure, etc., which is coupled to a proximal end light source. In some instances, the proximal end light source is a forward focused LED. Of interest are in such embodiments are bright LEDs, e.g., LEDs having a brightness of 100 mcd or more, such as 300 mcd or more, and in some instances 500 mcd or more, 1000 mcd or more, 1500 mcd or more. In some instances, the brightness ranges from 100 to 2000 mcd, such as 300 to 1500 mcd. The LED may be coupled with a forward focusing lens that is, in turn, coupled to the light conveyance structure.

In some instances, the proximal end LED may be coupled to the light conveyance structure in a manner such that substantially all, if not all, light emitted by the LED is input into the light conveyance structure. Alternatively, the LED and focusing lens may be configured such that at least a portion of the light emitted by the LED is directed along the outer surface of the elongated member. In these instances, the forward focused light emitting diode is configured to direct light along the outer surface of the elongated member. As such, light from the proximal end LED travels along the outer surface of the elongated member to the distal end of the elongated member.

In some instances, the tissue visualization devices of the invention are configured to reduce coupling of light directly from the integrated illuminator to the visualization sensor. In other words, the devices are structures so that substantially all, if not all, of the light emitted by the integrated illuminator at the distal end of the elongated structure is prevented from directly reaching the visualization sensor. In this manner, the majority, if not all, of the light that reaches the visualization sensor is reflected light, which reflected light is converted to image data by the visualization sensor. In order to substantially prevent, if not inhibit, light from the integrated illuminator from directly reaching the integrated visualization sensor, the device may include a distal end polarized member. By distal end polarized member is meant a structure or combination of structures that have been polarized in some manner sufficient to achieve the desired purpose of reducing, if not eliminating, light from the integrated illuminator directly reaching the integrated visualization sensor. In one embodiment, the light from an LED is polarized by a first polarizer (linearly or circularly) as it enters at lens or prism at the distal tip of the elongated member. A visualization sensor, such as CMOS sensor, also has a polarizer directly in front of it, with this second polarizer being complimentary to the first polarizer so that any light reflected by the outer prism surface into the visualization sensor will be blocked by this polarizer. Light passing through the first polarizer and reflected by the surrounding tissue will have random polarization, so roughly half of this light will pass through the second polarizer to reach the visualization sensor and be converted to image data. The distal end polarized member may be a cover lens, e.g., for forward viewing elongated members, or a prism, e.g., for off-axis viewing elongated members, such as described in greater detail below.

In some instances, the distal end of the elongated member includes an off-axis visualization module that is configured so that the visualization sensor obtains data from a field of view that is not parallel to the longitudinal axis of the elongated member. With an off-axis visualization module, the field of view of the visualization sensor is at an angle relative to the longitudinal axis of the elongated member, where this angle may range in some instances from 5 to 90°, such as 45 to 75°, e.g., 30°. The off-axis visualization module may include any convenient light guide which collects light from an off-axis field of view and conveys the collected light to the visualization sensor. In some instances, the off-axis visualization module is a prism.

Depending on the particular device embodiment, the elongated member may or may not include one or more lumens that extend at least partially along its length. When present, the lumens may vary in diameter and may be employed for a variety of different purposes, such as irrigation, aspiration, electrical isolation (for example of conductive members, such as wires), as a mechanical guide, etc., as reviewed in greater detail below. When present, such lumens may have a longest cross section that varies, ranging in some instances from 0.5 to 5.0 mm, such as 1.0 to 4.5 mm, including 1.0 to 4.0 mm. The lumens may have any convenient cross-sectional shape, including but not limited to circular, square, rectangular, triangular, semi-circular, trapezoidal, irregular, etc., as desired. These lumens may be provided for a variety of different functions, including as conveyance structures for providing access of devices, compositions, etc. to the distal end of the elongated member, as described in greater detail below. Such lumens may be employed as a "working channel".

In some embodiments, an integrated articulation mechanism that imparts steerability to at least the distal end of the elongated member or a component thereof is also present in the device, such that the elongated member is the elongated member is configured for distal end articulation. By "steerability" is meant the ability to maneuver or orient the distal end of the elongated member or component thereof as desired during a procedure, e.g., by using controls positioned at the proximal end of the device, e.g., on the hand-held control unit. In these embodiments, the devices include a steerability mechanism (or one or more elements located at the distal end of the elongated member) which renders the desired elongated member distal end or component thereof maneuverable as desired through proximal end control. As such, the term "steerability", as used herein, refers to a mechanism that provides a user steering functionality, such as the ability to change direction in a desired manner, such as by moving left, right, up or down relative to the initial direction. The steering functionality can be provided by a variety of different mechanisms. Examples of suitable mechanisms include, but are not limited to one or more wires, tubes, plates, meshes or combinations thereof, made from appropriate materials, such as shape memory materials, music wire, etc.

In some instances, the distal end of the elongated member is provided with a distinct, additional capability that allows it to be independently rotated about its longitudinal axis when a significant portion of the operating handle is maintained in a fixed position, as discussed in greater detail below. The extent of distal component articulations of the invention may vary, such as from -180 to +180°; e.g., -90 to +90°. Alternatively, the distal probe tip articulations may range from 0 to 360°, such as 0 to +180°, and including 0 to +90°, with provisions for rotating the entire probe about its axis so that the full range of angles is accessible on either side of the axis of the probe, e.g., as described in greater detail below. Rotation of the elongated member may be accomplished via any convenient approach, e.g., through the use of motors, such as described in greater detail below. Articulation mechanisms of interest are further described in published PCT Application Publication Nos. WO 2009029639; WO 2008/094444; WO 2008/094439 and WO 2008/094436. Specific articulation configurations of interest are further described in connection with the figures, below, as well as in United States Application Serial No. 12/422,176.

As summarized above, the internal tissue visualization devices of the invention further include a hand-held control unit to which the elongated member is operably connected. By "operably connected" is meant that one structure is in communication (for example, mechanical, electrical, optical connection, or the like) with another structure. The hand-held control unit is located at the proximal end of the elongated structure, and therefore at the proximal end of the device. As the control unit is hand-held, it is configured to be held easily in the hand of an adult human. Accordingly, the hand-held control unit may have a configuration that is amenable to gripping by the human adult hand. The weight of the hand-held control unit may vary, but in some instances ranges from .5 to 5 lbs, such as .5 to 3 lbs (1lbs = 0.454 Kg aprox). The hand-held control unit may have any convenient configuration, such as a hand-held wand with one or more control buttons, as a hand-held gun with a trigger, etc., where examples of suitable handle configurations are further provided below.

In some instances, the hand-held control unit may include a monitor. By monitor is meant a visual display unit, which includes a screen that displays visual data in the form of images and/or text to a user. The screen may vary, where a screen type of interest is an LCD screen. The monitor, when present, may be integrated or detachable from the remainder of the hand-held control unit. As such, in some instances the monitor may be an integrated structure with the hand-held control unit, such that it cannot be separated from the hand-held control unit without damaging the monitor in some manner. In yet other embodiments, the monitor may be a detachable monitor, where the monitor can be attached to and separated from the hand-held control unit, as desired, without damaging the function of the monitor. In such embodiments, the monitor and hand-held control unit may have a variety of different mating configurations, such as where the hand-held control unit includes a hole configured to receive a post of the monitor, where the monitor has a structure that is configured to snap onto a receiving structure of the hand-held control unit, etc. The monitor, when present will have dimensions sufficient for use with the hand-held control unit, where screen sizes of interest may include 10 inches or smaller, such es or smaller, e.g., 5 inches or smaller, e.g., 3.5 inches, etc.

Data communication between the monitor and the remainder of the hand-held control unit may be accomplished according to any convenient configuration. For example, the monitor and remaining components of the hand-held control unit may be connected by one or more wires. Alternatively, the two components may be configured to communication with each other via a wireless communication protocol. In these embodiments, the monitor will include a wireless communication module.

In some embodiments, the distal end of the elongated member is rotatable about its longitudinal axis when a significant portion of the hand-held control unit is maintained in a fixed position. As such, at least the distal end of the elongated member can turn by some degree while the hand-held control unit attached to the proximal end of the elongated member stays in a fixed position. The degree of rotation in a given device may vary, and may range from 0 to 360°, such as 0 to 270°, including 0 to 180°. Rotation, when present, may be provided by any convenient approach, e.g., through use of motors.

Devices of the invention may be disposable or reusable. As such, devices of the invention may be entirely reusable (e.g., be multi-use devices) or be entirely disposable (e.g., where all components of the device are single-use). In some instances, the device can be entirely reposable (e.g., where all components can be reused a limited number of times). Each of the components of the device may individually be single-use, of limited reusability, or indefinitely reusable, resulting in an overall device or system comprised of components having differing usability parameters.

Of interest are devices in which the hand-held control unit is reusable. In such devices, the elongated member is configured to be detachable from the hand-held control unit. As the elongated member is configured to be readily separable from the hand-held control unit without in any way damaging the functionality of the hand-held control unit, such that the hand-held control unit may be attached to another elongated member. As such, the devices are configured so that the hand-held control unit can be sequentially operably attached to multiple different elongated members. Of interest are configurations in which the elongated member can be manually operably attached to a hand-held control unit without the use of any tools. A variety of different configurations may be employed, e.g., where the proximal end of the elongated member engages the hand-held control unit to provide an operable connection between the two, such as by a snap-fit configuration, an insertion and twist configuration, etc. In certain configurations, the hand-held control unit has a structure configured to receive the proximal end of the elongated member.

In some instances, the hand-held control unit may be re-used simply by wiping down the hand-held control unit following a given procedure and then attaching a new elongated member to the hand-held control unit. In other instances, to provide for desired sterility to the hand-held control unit, the device may include a removable sterile covering attached to the proximal end of the elongated member that is configured to seal the hand-held control unit from the environment. This sterile covering (e.g., in the form of a sheath as described in greater detail below) may be a disposable sterile handle cover that uses a flexible bag, a portion of which is affixed to and sealed to the proximal end of the disposable elongated member. Where desired, the sterile covering may include an integrated clear monitor cover, which may be rigid and configured to conform to the monitor screen. In some instances, the cover may be configured to provide for touch screen interaction with the monitor. As indicated above, the hand-held control unit may include a manual controller. In such instances, the sterile covering may include a flexible rubber boot for mechanical controller sealing, i.e., a boot portion configured to associated with the manual controller. In addition, the sterile covering may include a seal at a region associated with the proximal end of the hand-held control unit. In these instances, the open side of sterile cover prior to use may be conveniently located at the proximal end. Following positioning of the cover around the hand-held control unit, the open side may be mechanically attached to the handle and closed by a validated sealing method. The sterile cover of these embodiments is configured such that when employed, it does not inhibit handle controls or elongated structure and monitor actuation.

In addition to the distal end integrated visualization sensor, e.g., as described in greater detail above, devices of the invention may include a distal end integrated non-visualization sensor. In other words, the devices may include one or more non-visualization sensors that are integrated at the distal end of the elongated member. The one or more non-visualization sensors are sensors that are configured to obtain non-visual data from a target location. Non-visual data of interest includes, but is not limited to: temperature, pressure, pH, elasticity, impedance, conductivity, distance, size, etc. Non-visualization sensors of interest include those configured to obtain one or more types of the non-visual data of interest. Examples of sensors that may be integrated at the distal end include, but are not limited to: temperature sensors, pressure sensors, pH sensors, impedance sensors, conductivity sensors, elasticity sensors, etc. Specific types of sensors include, but are not limited to: thermistors, strain gauges, membrane containing sensors, MEMS sensors, electrodes, light sensors, etc. The choice of a specific type of sensor will depend on the nature of the non-visual data of interest. For example, a pressure sensor can detect the force applied to a target tissue as it is deformed to determine the elastic modulus of the target tissue. A temperature sensor can be employed to detect locally elevated temperatures (which can be used to differentiate different types of tissue, such as to different normal and tumor tissue (where tumors exhibit increased bloodflow and therefore a higher temperature)). A properly collimated laser beam could be used to determine the distance to objects in the device field of view or the length scale of objects in the device field of view. When present, the integrated non-visualization sensor or sensors may be configured to complement other distal end components of the devices, so as to minimize any impact on the outer dimension of the distal end, e.g., in ways analogous to those described above in connection with integrated illumination elements.

In some instances, the devices include a tissue modifier. Tissue modifiers are components that interact with tissue in some manner to modify the tissue in a desired way. The term modify is used broadly to refer to changing in some way, including cutting the tissue, ablating the tissue, delivering an agent(s) to the tissue, freezing the tissue, etc. As such, of interest as tissue modifiers are tissue cutters, tissue ablators, tissue freezing/heating elements, agent delivery devices, etc. Tissue cutters of interest include, but are not limited to: blades, liquid jet devices, lasers and the like. Tissue ablators of interest include, but are not limited to ablation devices, such as devices for delivery ultrasonic energy (e.g., as employed in ultrasonic ablation), devices for delivering plasma energy, devices for delivering radiofrequency (RF) energy, devices for delivering microwave energy, etc. Energy transfer devices of interest include, but are not limited to: devices for modulating the temperature of tissue, e.g., freezing or heating devices, etc. In some embodiments, the tissue modifier is not a tissue modifier that achieves tissue modification by clamping, clasping or grasping of tissue such as may be accomplished by devices that trap tissue between opposing surfaces (e.g., jaw-like devices). In these embodiments, the tissue modification device is not an element that is configured to apply mechanical force to tear tissue, e.g., by trapping tissue between opposing surfaces.

In some instances, the tissue modifier is a low-profile tissue modifier, such as a low-profile biopsy tool or a low-profile cutter. Such low-profile tissue modifiers are include tissue cutting structure positioned at the distal of the elongated member. Because the biopsy or cutting tool is low-profile, its presence at the distal end of the elongated member does not substantially increase the outer diameter of the elongated member. In some instances, the presence of the low-profile biopsy tool increase the outer diameter of the elongated member by 2 mm or less, such as 1.5 mm or less, including 1 mm or less. The configuration of the low-profile biopsy tool may vary. In some instances, the low-profile biopsy tool comprises an annular cutting member concentrically disposed about the distal end of the elongated member and configured to be moved relative to the distal end of the elongated member in a manner sufficient to engage tissue. The annular cutting member may or may not be configured as a complete ring structure, where the ring structure is movable in a longitudinal manner relative to the distal end of the elongated member (such that it may be moved along the elongated member towards and away from the proximal end of the elongated member). The distal edge of the ring structure may be movable some distance beyond the distal end of elongated member, where this distance may vary and in some instances is 10 mm or less, such as 5 mm or less, including 3 mm or less. The distal edge of the ring structure may be sharp in order to penetrate tissue, and may include one or more tissue retaining structures, such as barbs, hooks, lips, etc., which are configured to engage the tissue and stably associate the engaged tissue with the ring structure, e.g., when the ring structure is moved longitudinally along the elongated member towards the proximal end. Also of interest are cutting tools, e.g., as described

In some instances, the distal end integrated visualization sensor is present as an RF-shielded visualization module. As the visualization sensor module of these embodiments is RF-shielded, the visualization sensor module includes an RF shield that substantially inhibits, if not completely prevents, an ambient RF field from reaching and interacting with circuitry of the visualization sensor. As such, the RF shield is a structure which substantially inhibits, if not completely prevents, ambient RF energy (e.g., as provided by a distal end RF electrode, as described in greater detail blow) from impacting the circuitry function of the visualization sensor.

Visualization sensor modules of devices of the invention include at least a visualization sensor. In certain embodiments, the devices may further include a conductive member that conductively connects the visualization sensor with another location of the device, such as a proximal end location. Additional components may also be present in the visualization sensor module, where these components are described in greater detail below.

The RF shield of the visualization sensor module may have a variety of different configurations. The RF shield may include an enclosure element or elements which serve to shield the circuitry of the visualization sensor from an ambient RF field. In some instances, the RF shield is a grounded conductive enclosure component or components which are associated with the visualization sensor, conductive member and other components of the visualization sensor module. In some instances, the visualization sensor of the visualization sensor module is present in a housing, where the housing may include a grounded outer conductive layer which serves as an RF shield component. In these instances, the RF shield is an outer grounded conductive layer. The conductive enclosure or enclosures of the RF-shielded visualization sensor module may be fabricated from a variety of different conductive materials, such as metals, metal alloys, etc., where specific conductive materials of interest include, but are not limited to: copper foils and the like. In certain instances, the RF shield is a metallic layer. This layer, when present, may vary in thickness, but in some instances has a thickness ranging from 0.2mm to 0.7mm, such as 0.3mm to 0.6mm and including 0.4mm to 0.5mm. Additional details regarding RF-shielded visualization modules may be found in United States application serial no. 12/437,865.

In some instances, the may include a collimated laser configured to emit collimated laser light from a distal region of the elongated member, such as the distal end of the elongated member. The collimated laser components of these embodiments may be configured for use for a variety of purposes, such as but not limited to: anatomical feature identification, anatomical feature assessment of sizes and distances within the field of view of the visualization sensor, etc.

The devices of the invention may be fabricated using any convenient materials or combination thereof, including but not limited to: metallic materials such as tungsten, stainless steel alloys, platinum or its alloys, titanium or its alloys, molybdenum or its alloys, and nickel or its alloys, etc; polymeric materials, such as polytetrafluoroethylene, polyimide, PEEK, and the like; ceramics, such as alumina (e.g., STEATITE™ alumina, MAECOR™ alumina), etc.

In some instances, the devices may include a stereoscopic image module. By stereoscopic image module is meant a functional module that provides a stereoscopic image from image data obtained by the device. As such, the module provides a user via the monitor with the perception of a three-dimensional view of an image produced from the image data obtained by the device. The module is described in terms of "images", and it should be understood that the description applies equally to still images and video.

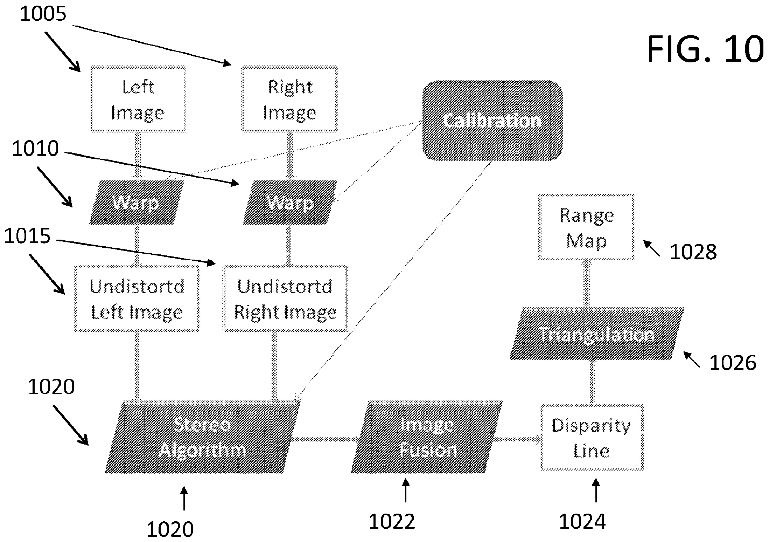

Where the device includes a stereoscopic image module, the device may include two or more distinct visualization sensors (e.g., CMOS cameras as reviewed above) or a single visualization sensor via which the image data is collected and employed by the stereoscopic image module to provide the stereoscopic image. Where the elongated member includes first and second visualization sensors, the stereoscopic imaging module is configured to process imaged data provided by the first and second visualization sensors to produce the stereoscopic image. In such embodiments, any convenient stereoscopic image processing program may be employed. FIG. 10 illustrates a block flow diagram of a technique to produce stereoscopic images from image data, according to one embodiment. Left and right image data are obtained (as represented by blocks 1005), either sequentially from a single visualization sensor that is moved from a first position to a second position or, if two visualization sensors are present, sequentially or simultaneously. The left and right Image data account for the different locations and perspectives associated with each respective position of the same visualization sensor or respective positions of the two distinct visualization sensors. The image data for the first and second images may include distortions, and an algorithm may be employed, for example, in which the left and right image data are first warped as shown via a calibration element to remove lens distortion, as represented by blocks 1010. Any convenient algorithm may be employed. Algorithms of interest include those described in "Geometric Calibration of Digital Cameras through Multi-view Rectification" by Luca Lucchese (Image and Vision Computing, Vol. 23, Issue 5, May 2005, pp. 517-539); and Levenberg-Marquardt algorithm, "Correction of Geometric Lens Distortion through Image Warping" by Lucchese (ISPA 2003, Proceeding of the 3rd International Symposium on Image and Signal Processing and Analysis, 18-20 Sept. 2003, Vol. 1, pp. 516-521). The resultant undistorted left and right images, represented by blocks 1015, are then processed with stereo and image fusion algorithms to construct a stereoscopic image, as represented at blocks 1020,1022,1024,1026,1028. Any convenient stereo and image fusion algorithms may be employed, such as but not limited to those described in: "Scene Reconstruction from Multiple Cameras" by Richard Szeliski (Microsoft Vision Technology Group; see also, http://research.microsoft.com/pubs/75687/Szeliski-ICIP00.pdf; "A parallel matching algorithm for stereo vision", by Y. Nishimoto and Y. Shirai (IJCAI-1985-Volume 2, pg. 977; see also, http://ijcai.org)/Past%20Proceedings/IJCAI-85-VOL2/PDF/059.Ddf): "Image Fusion Using Wavelet Transform", by Zhu Shu-long (Institute of Surveying & Mapping; Commission IV, Working Group IV/7; see also, http://www.isprs.org/commission4/proceedings02/pdfpapers/162.pdf); "Disparity field and depth map coding for multiview 3D image generation", by D. Tzovaras (Image Communication, Signal Processing; 1998, vol. 11, n°3, pp. 205-230); etc.

Stereo algorithms compute range information to objects seen by the visualization sensors by using triangulation. Objects seen at different viewpoints will result in the object at different locations in the image data for the first and second visualization sensors. The disparity, or image difference, is used in determining depth and range of objects. Corresponding pixel points within the image data for the first and second visualization sensors may be identified and used in the determination of disparity line, as represented by block 1024. Because the first and second visualization sensors are at different locations and hence have different perspectives, the same object present in image data for the first and second visualization sensor may be at different pixel coordinate locations. Triangulation may be implemented, as represented by block 1026, based on geometry associated with the locations of the first and second visualization sensors may be used to determine depth and range of objects seen by the visualization sensors. Triangulation computations are applied to derive range data, and the resultant range (or depth) map can be overlayed on the image display, as desired. This is represented at block 1028 in FIG. 10. Stereoscopic images taking into account three-dimensional depth information can thus be reconstructed from image data from the first and second visualization sensor.

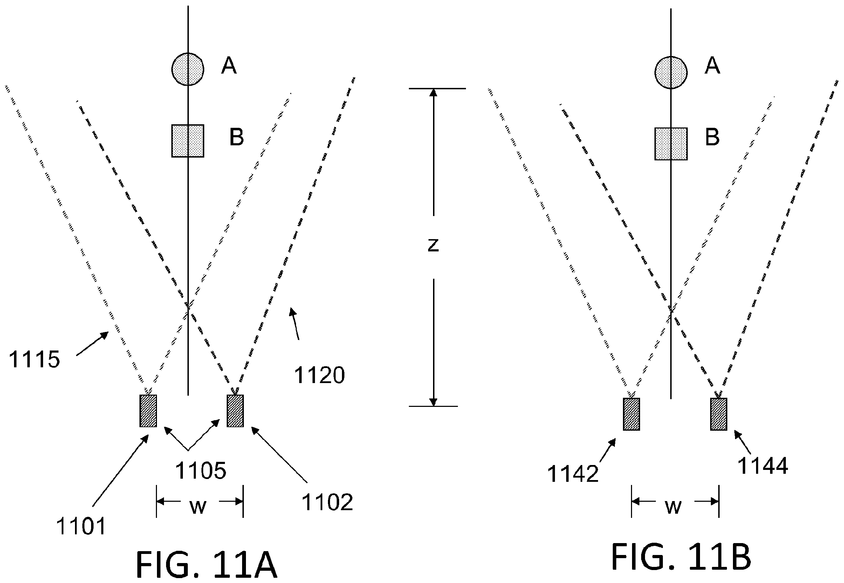

FIGS. 11B illustrates slightly offset visualization positions, according to certain embodiments. FIG. 11B illustrates two visualization sensors, i.e., 1142 for a first view of objects A and B and 1144 for a second view of objects A and B. The depth and range of the object is found in a similar manner as for FIG. 11A, as described in more above.

Further details regarding aspects of stereoscopic image modules that employ image data obtained by two or more distinct visualization sensors may be found in United States application serial no 12/269,770.

Also of interest are stereoscopic image modules that are configured to provide a stereoscopic image from data obtained by a single image sensor. In such embodiments, the image sensor is configured to provide to the stereoscopic image module consecutive offset image data of the target tissue location, which consecutive offset image data are then employed by the stereoscopic image module to provide the desired stereoscopic image. By consecutive offset image data is meant image data that includes at least data from a first view of a target tissue location and data from a second view of the same target location, where the second view is offset from the first view. The second view may be offset from the first view by any convenient distance, for example 1 mm or less, including .5 mm or less; The first and second offset views may be obtained using any convenient approach. In one approach, the single visualization sensor is moved from a first position to a second position in order to obtain the desired offset image data. The single visualization sensor may be moved from the first to the second positions using any convenient manner, e.g., by a mechanical element that physically moves the sensor from the first to the second position. In yet other embodiments, the desired offset views may be obtained with a single visualization sensor operatively coupled to an optical guide system (which may include one or more of lenses, mirrors, filters, etc.) configured to provide the desired first and second offset views. For example, the first and second offset views may be provided to the single visualization sensor by including a first and second lens systems which alternately convey image data to the visualization sensor. The offset views may also be provided, for example, by including a single lens system with mirrors configured to provide the lens with two or more different views. The frequency with which the first and second offset views are obtained may vary, where in some instances the frequency may range from 1 to 30 frames/sec, such as 1 to 15 frames/sec. Various systems may be implemented to provide multiple views with a single camera. Systems of interest include, but are not limited to, those described in: "Scalable Multi-view Stereo Camera Array for Real World Real-Time Image Capture and Three Dimensional Displays" by S. Hill (Massachusetts Institute of Technology, Program in Media Arts and Sciences School of Architecture and Planning; May 7, 2004; see also, http://web.media.mit.edu/∼vmb/papers/hillms.pdf); "Single Camera Stereo Using Planar Parallel Plate" by Chunyu Gao, et al. (Beckman Institute, University of Illinois at Urbana-Champaign; see also, http://vision.ai.uiuc.edu/newpubs/Stereo PPP Gao.pdf): and, "3-D Reconstruction Using Mirror Images Based on a Plane Symmetry Recovering Method" by Mitsumoto, H., et al. (IEEE Transaction on Pattern Analysis and Machine Intelligence; Vol. 14; Issue No. 9, September 1992, pp. 941-946).

FIG. 11A illustrates a single visualization sensor 1105 which is moved to two different positions (1101 and 1102) to sequentially obtained image data, which sequentially obtained image data is employed by a stereoscopic image module to produce a stereoscopic image of objects A and B. The first and second visualization positions 1101 and 1102 are at an offset width W from one another, which may vary, ranging in some instances from 1 mm or less, such as .5 mm or less. Objects A and B located at a focal plane distance Z are seen at different perspectives for the first and second positions (shown by dotted lines 1115, 1120, respectively). The difference in viewing perspectives is reflected in the image data obtained by the single image sensor from the first and second positions. As shown, first visualization sensor 1105 sees objects A & B off to the right of center when in position 1101 and sees objects A and B off to left of center when in position 1102. The disparity between the two views is used to determine depth and range of objects A and B.

The stereoscopic image module may be implemented in a video processor module configured to receive image data obtained by the one or more visualization sensors. The stereoscopic image module processes the image data to provide stereoscopic image data for display on a display.

In certain embodiments, devices of the invention include an image recognition module. Image recognition modules of interest are those that are configured to receive image data and compare the received image data with a reference that includes at least one of color descriptor data and anatomical descriptor data to make a determination as to whether an alert signal should be generated. The term "reference" is used herein to refer to data in any format, e.g., saved as one or more image files, etc., that is for one or more reference images, e.g., where the data can be used by an appropriate processor to produce one or more reference images. As such, a reference includes at least a first set of reference image data for a first reference image. In some instances a reference also includes a second set of reference image data for a second reference image. In such embodiments, a reference may include sets of reference image data for multiple reference images, e.g., 2 or more, 5 or more, 10 or more, 25 or more, 50 or more, 100 or more, 1000 or more, 1500 or more, 2000 or more, 5000 or more, 10, 000 or more etc., reference images.

Reference images are predetermined images of a region of interest. As the reference images are predetermined, they are images that have been produced independently of the image data that is received by the image processing module. In some instances, the reference images are images that exist prior to obtainment of the image data that is received by the image processing module. The reference images may be images that are obtained from the same subject (e.g., person) that is being visualized during a given procedure (e.g., where the reference images were obtained from the subject prior to a given procedure) or from a different subject (e.g., person). Alternatively, the reference images may be produced de novo, such that they are not produced from image data obtained from any actual subject but instead are designed, e.g., by using manual or computer assisted graphic protocols.

Reference images that make up the reference may differ from each other in a number of ways. For example, any two given reference images may be images of regions of interest of different internal tissue locations. In such a reference, the reference may include first and second pre-determined images that differ from each other with respect to a pre-determined internal tissue location. For example, the reference may include images of at least a first tissue location and a second tissue location. The first and second tissue locations may be locations that a given device may be expected to image during a given procedure, such as during a surgical procedure. In some instances, the reference includes multiple images of different locations that a given visualization sensor should image during a given procedure if the procedure is performed correctly. The reference may also include images of different tissue locations that a visualization sensor should not see during a given procedure, e.g., images of tissue locations that should not be viewed by the sensor if the given procedure of interest is being performed correctly. Accordingly, some references may include multiple images that track the location of a device when correctly and incorrectly positioned during an entire procedure, such as an entire surgical procedure.

The sets of image data in the reference may include one or more color descriptor data and anatomical descriptor data. By color descriptor data is meant data which is based on the particular color of a given internal tissue site and components thereof. For example, an internal tissue site may include one or more tissues that each has a distinct color. For example, different tissues such as muscle, nerve, bone, etc., may have different colors. This distinct color may be present in the reference image as color descriptor data, and employed by the image processing module. By anatomical descriptor data is meant data which is based on the particular shape of one or more tissue structures at the internal tissue site. For example, different tissues such as muscle, nerve, bone, etc., have different shapes. These different shapes are present in the image data as anatomical descriptor data.

As summarized above, the image recognition module compares received image data of an internal tissue site (e.g., obtained during a given procedure of interest) with the reference. The comparison performed by the image recognition module may be achieved using any convenient data processing protocol. Data processing protocols that may be employed in this comparison step may compare the received image data and reference based on color descriptor data and/or anatomical descriptor data. Data comparison protocols of interest include, but are not limited to: mean absolute difference between the descriptors of data and stored values such as mean color intensity, and, the degree of correlation between principle axis of the structure and stored values.

In performing this comparison step, the image recognition module may be configured to automatically select the appropriate images from a reference to compare against the received image data. In some instances, the image recognition module is configured to compare the received image data with the reference by selecting an appropriate set of reference image data based on a determined positional location of the device. For example, the image recognition module may obtain positional information about the device (e.g., as may be obtained from sensors on the device or manually input and associated with a given image) and then select reference images that are for the same positional location as the device when the device obtained the image data being received. Alternatively, the image recognition module may automatically select appropriate sets of image data based on similarity parameters. For example, the image recognition module may automatically select the most similar sets of image data from the reference to use in the comparison step.

The image recognition module compares the received image data with the reference in order to determine whether an alert signal should be generated. In other words, the output of the image recognition module is a decision as to whether an alert signal should be generated. If an image recognition module determines that an alert signal should be generated, it may generate the alert signal or instruct a separate module of the system to produce an alert signal.

The alert signal, when generated, may vary depending on the nature of the system. An alert signal may be a warning signal about a given system parameter or a signal that confirms to an operator of the system that a given system parameter of interest is acceptable. In some embodiments, an alert signal may include functional information about a device. For example, in these embodiments an alert signal may include information that a given device is functioning properly. In some embodiments, an alert signal may include positional information about a device. For example, an alert signal may include information as to whether or not a given device is correctly spatially positioned. In these embodiments, the alert signal may contain information that a tissue modifier of the device is contacting non-target tissue, such that the tissue modifier is not correctly spatially positioned.

The system may be configured to employ an alert signal in a variety of different ways. The system may be configured to provide the alert signal to a user of the system, e.g., via an alert signal output of the system. In addition or alternatively, the system may be configured to automatically modulate one or more operational parameters of the system based on the generation of an alert signal. For example, where the image processing module determines that a tissue modifier is contacting non-target tissue and therefore generates an alert signal, the alert signal may automatically modulate operation of the tissue modifier, e.g., by turning it off. In some instances, the alert signal may automatically shut the system down.

Further details regarding image recognition modules are provided in U.S. application serial no. 12/437,186.

The stereoscopic module and image recognition modules, e.g., as described above, may be implemented as software, e.g., digital signal processing software; hardware, e.g., a circuit; or combinations thereof, as desired.

In some embodiments, the devices may include a conveyance structure configured to convey an item between the distal end of the elongated member and an entry port positioned at a proximal end of the device, e.g., associated with the proximal end of the elongated member or associated with the hand-held control unit. This conveyance structure may have any convenient configuration, where in some instances it is a "working channel" disposed within the elongated member. When present as a working channel, the channel may have an outer diameter that varies, and in some instances has an outer diameter of 3 mm or less, such as 2 mm or less and including 1 mm or less. The conveyance structure may be configured to transport items, e.g., fluids, medicines, devices, to an internal target site or from an internal target site. As such, the proximal end entry port of the conveyance structure may vary, and may be configured to be operably coupled to a variety of different types of components, such as but not limited to: aspiration units, fluid reservoirs, device actuators, etc.

As indicated elsewhere, devices of the invention may be configured for wireless data transmission, e.g., to provide for one or more of: transmission of data between various component of the device, transmission of data between components of the device and another device, such as hospital information system, separate monitor, etc. Any convenient wireless communication protocol may be employed, where in some instances wireless communication is implemented as one or more wireless communication modules.

A video processor module may be present and be configured to control the one or more distinct visualization sensors by sending camera control data to a camera module including the visualization sensor(s). The video processor may also be configured to receive sensor data from one ore more sensors and/or tools; and further, may be configured to control the sensors and/or tools by sending sensor control data to a sensor module including the one or more sensors and/or tools. The various sensors may include, but are not limited to, sensors relating to pressure, temperature, elasticity, ultrasound acoustic impedance, laser pointer to identify and/or measure difference to sensors, etc. The various tools may include, but are not limited to, a measurement scale, teardrop probe, biopsy probe, forceps, scissors, implant device, IR lighting, ultrasound measurement device, cutting tool, etc. Depending on the specific application and sensor/tool implemented, sensor data may also be included with the image data for processing by the stereoscopic image module, in order to provide the stereographic images.

In certain instances, the devices of the invention include an updatable control module, by which is meant that the devices are configured so that one or more control algorithms of the device may be updated. Updating may be achieved using any convenient protocol, such as transmitting updated algorithm data to the control module using a wire connection (e.g., via a USB port on the device) or a wireless communication protocol. The content of the update may vary. In some instances, a hand-held control unit is updated to configure the unit to be used with a particular elongated member. In this fashion, the same hand-held control units may be employed with two or more different elongated members that may differ by function and have different components. In some instances, the update information may be transmitted from the particular elongated member itself, such that upon operable connection of the elongated member to the hand-held control unit, update information is transferred from the elongated member to the hand-held control unit that updates the control module of the hand-held control unit such that it can operate with that particular elongated member. The update information may also include general functional updates, such that the hand-held control unit can be updated at any desired time to include one or more additional software features and/or modify one or more existing programs of the device. The update information can be provided from any source, e.g., a particular elongated member, the internet, etc.

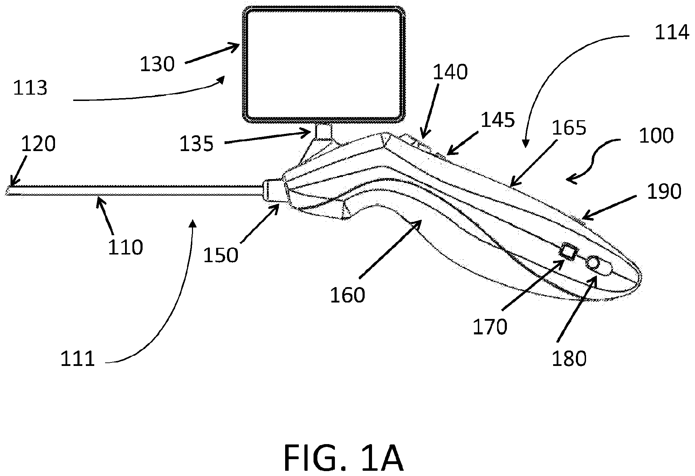

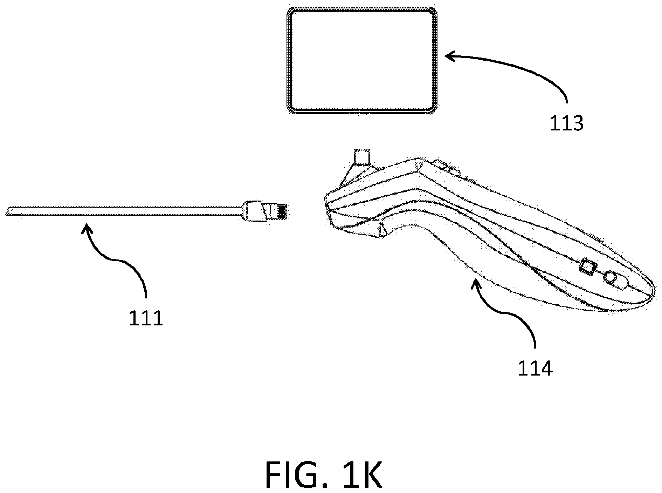

Turning now to the figures, FIGS. 1A-1K, illustrate one embodiment a self-contained, portable diagnostic imaging device of the invention. The hand-held, self-contained, portable diagnostic imaging device 100 illustrated in these figures includes a hand piece 114 and a removably attached elongated member 111 having a distal end integrated CMOS sensor, which is referred to herein as a "probe piece." See FIG. 1K.

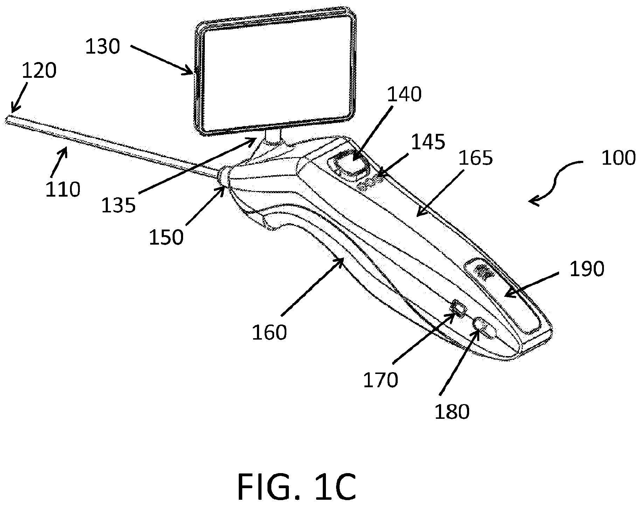

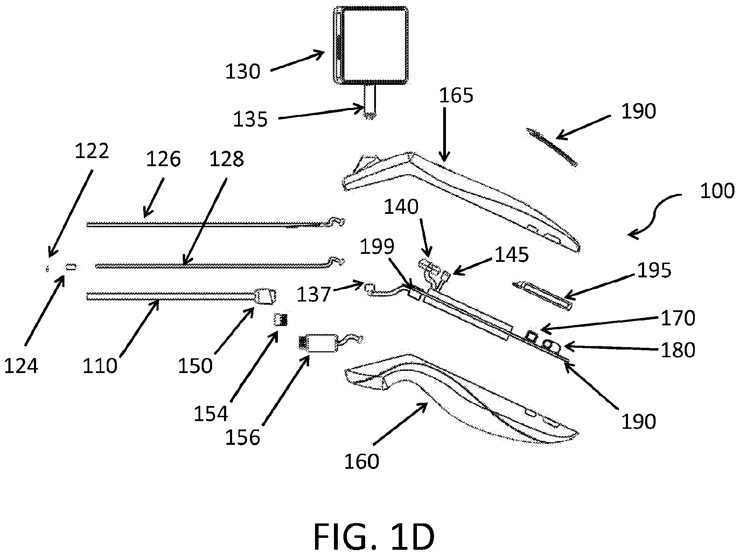

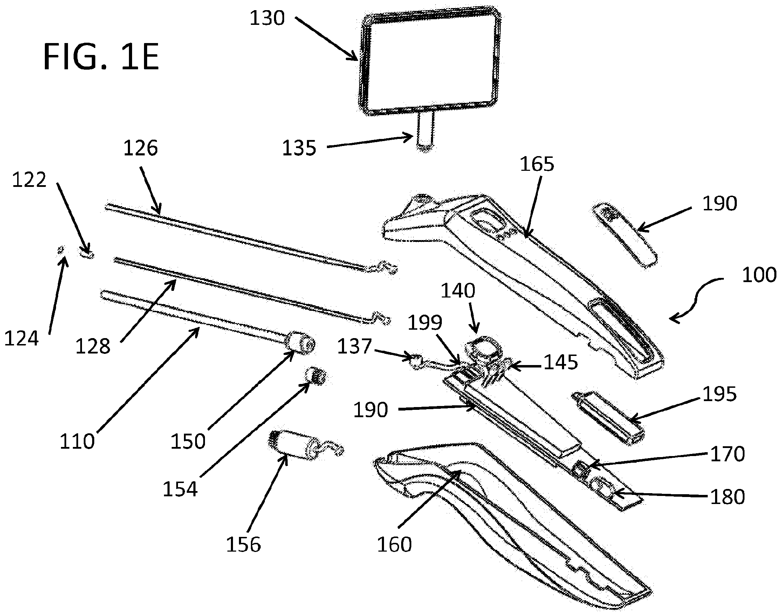

From an external view, the probe piece, as shown in FIG. 1A and 1C, includes a distal tip 120, an elongated tubular structure 110, and a mechanical connector 150 to the hand piece. The hand piece, from an external view, as shown in FIG. 1A and 1C, includes a rotatable and removable monitor unit 113 made up of a monitor 130 and a monitor mount 135 that may be attached to either the monitor housing or the top part of the hand piece depending on the embodiment, a single port 170, such as a USB port, for use as an input for programming or as an output for video and still images, an on/off switch 180 for managing power input to the device, a top cover 165, a bottom cover 160, switches for image capture and data transfer and control 145, and a switch for controlling the rotation of the probe piece 140. This switch 140 generally has three positions for controlling the motor rotation, one position to rotate the motor clockwise, one position to rotate the motor counterclockwise, and a position in the center that is neutral. Lastly, as shown in FIGS. 1D and 1E, there is a battery door 190 for the purpose of accessing the battery 195.

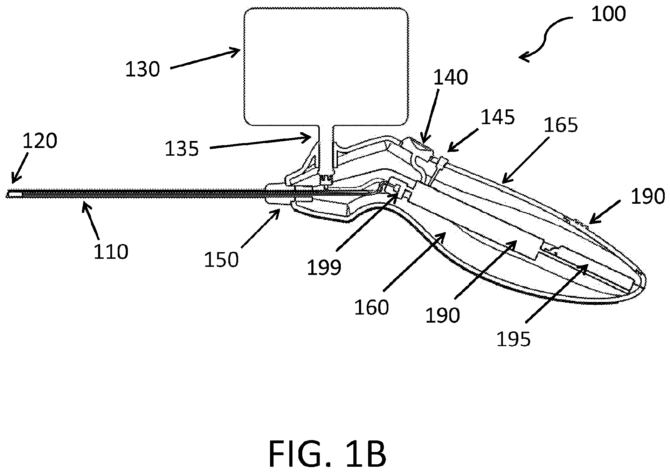

Internally viewed, the device additionally contains a battery 195 that may be rechargeable, an electronic control board 190, and connectors 199 for all electrical and optical components of the device, to and from the electronic control board 190, as shown in FIG. 1B.

Within the distal tip 120 of the probe piece, as shown in FIGS. 1D and 1E, is a lens 122, such as a prism lens, or a flat lens (e.g., cover glass), and a CMOS visualization sensor (referred to herein as a camera)124. Within the elongated structure portion 110 of the probe piece is a wire 128 for electrically connecting the camera 124 to a connector 199 on the electronics board 190. Also, an illuminator126 is arranged within the probe piece so as to provide lighting at the distal tip 120, and is connected to the electronic control board 190 at the connectors 199.

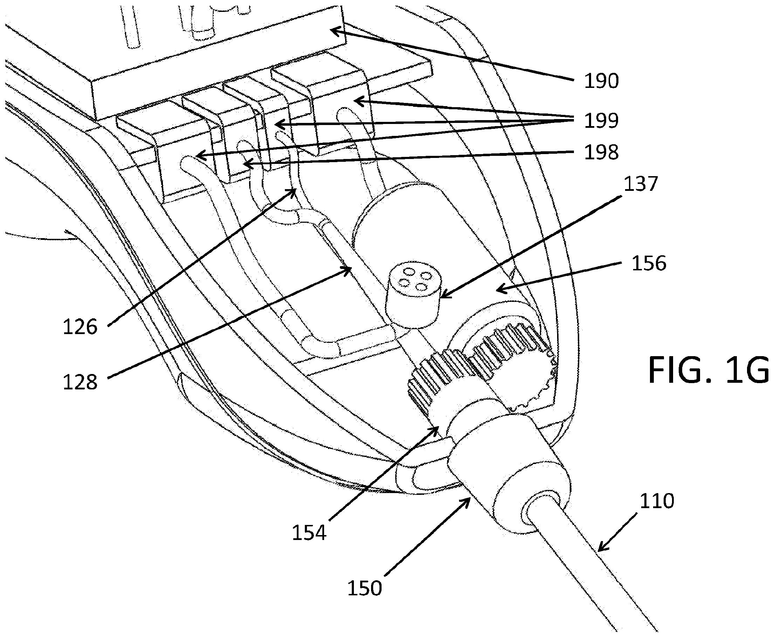

Also within the hand piece, in the present embodiment of the invention as shown in FIGS. 1D, 1E and 1G, is a geared motor 156. Geared motor 156 is connected to the probe piece via a geared intermediary piece 154. The connection between the geared motor 156 and the intermediary piece 154 of the probe piece is oriented in such a way as to allow for the rotation of the probe piece both counterclockwise and clockwise. The connector 150 linking the probe piece to the hand piece does not rotate with the intermediary piece 154.

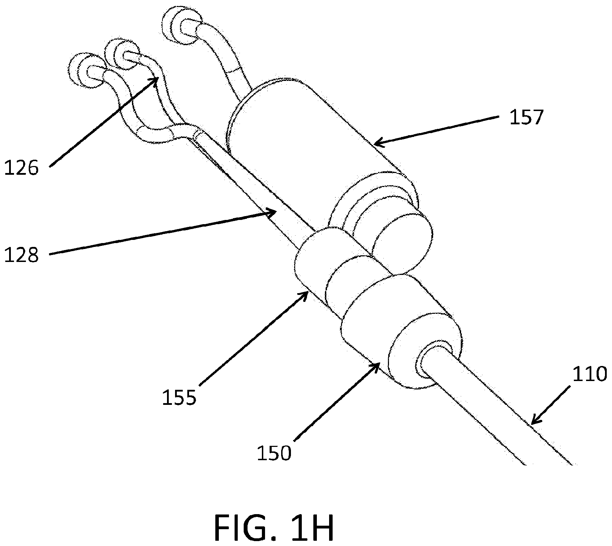

In another embodiment, as shown in FIG. 1H, there may be a frictional and rotational connection accomplished between the probe piece and the motor 157 by an intermediary piece 155, for example, a rubber to rubber contact connection. Both the motor 157 and the intermediary piece 155 are oriented in such a way as to allow for the rotation of the probe piece both counterclockwise and clockwise. The connector 150 linking the probe piece to the hand piece does not rotate with the intermediary piece 155.

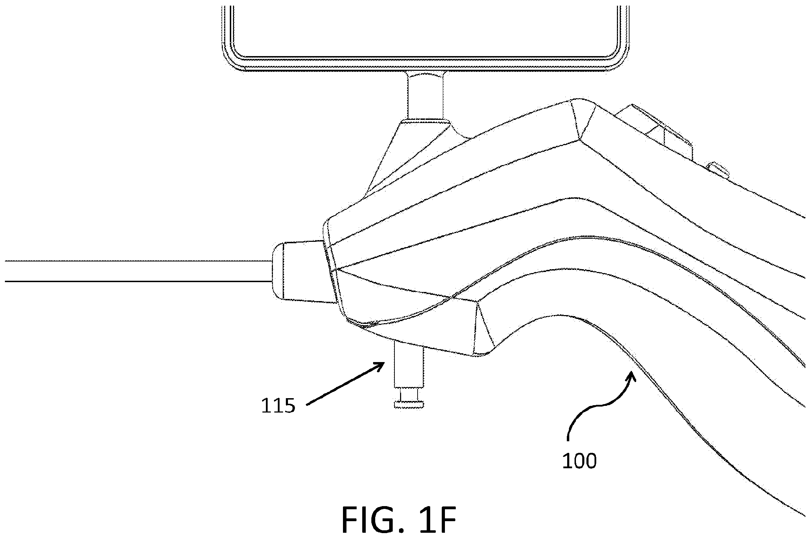

Lastly, referring to FIG. 1E and 1F, within the hand piece, there is a connector 137 for electrically coupling the monitor mount 135 to the electronic board 190. The connector 137 is configured to allow for the rotation of the monitor mount 135, and thus the monitor 130 connected to the monitor mount 135, without binding, breaking or kinking of the connector 137 or the associated wiring that connects the connector 170 to the electronic board 190.

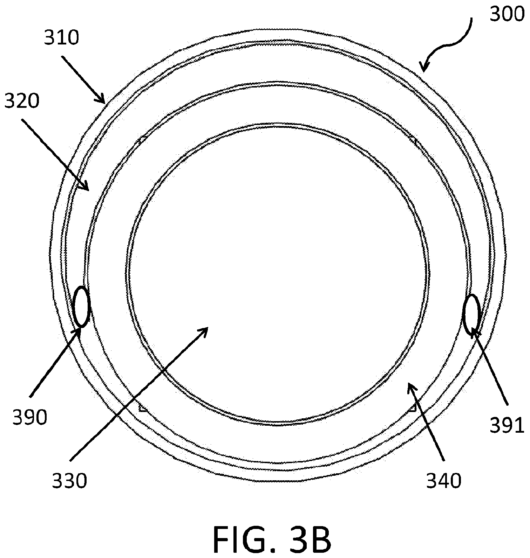

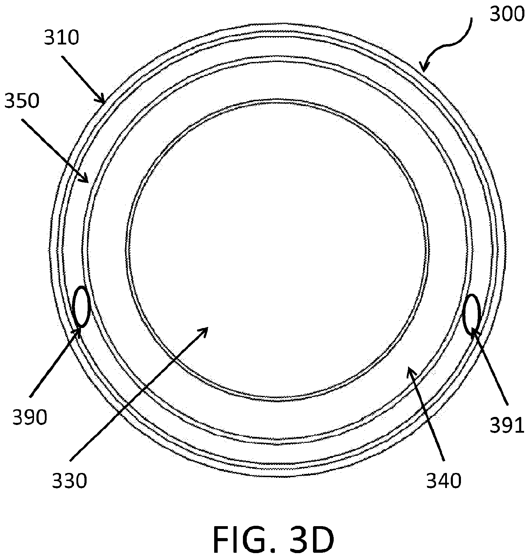

In another embodiment of the invention, the portable diagnostic imaging system 100 may include an element to transport material, medicine and implants to and from a point external to the hand piece and external to the distal tip 120 of the probe piece, e.g., a lumen configured as a working channel. As shown in FIG. 1F, there is a port connection 115, such as a luer connector for connecting to other luer connectors, for example a barbed connector for connecting to tubing, like a compression connector for connection to tubing. This port connector 115 may be located and protrude from either external half of the hand piece 165 and 160, and at any location convenient to the use of the device. Internal to both the hand piece and the probe piece is a conduit that connects the port 115 to a port 391, as shown in FIGS. 3B and 3D located at the very distal end of the distal tip 120 of the probe piece whereby a material, medicine or implant may be delivered from the hand piece 100. In another embodiment, the material, medicine or implant, may be aspirated into the port 391 at the distal tip 120 of the probe piece, and be transported through a conduit within the probe piece and hand piece, exiting through the port 115 located on the hand piece.

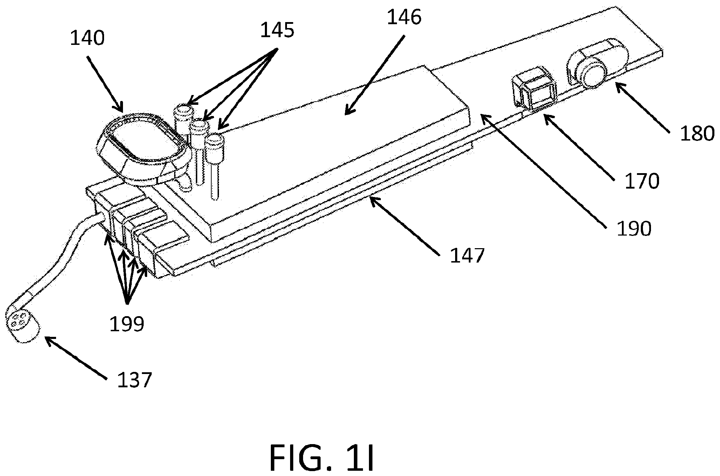

As mentioned above, devices of the invention may include an electronic board 190. FIG. 1I shows one embodiment of an electronic board 190 and its associated components. Generally speaking, one group of components that the electronic board 190 has electrically attached to it are electronic components of the control circuitry represented as blocks 146 and 147. In the example of FIG. 11, there are two locations for electronic components 146 and 147 on the electronic board 190, but there may only be required, in other embodiments of the invention, electronic components located on one side or the other of the electronic board 190, and not necessarily to the footprint of the electronic components 146,147 as suggested in FIG. 1I.

Another item that is electrically attached to the electronic board 190 is an electrical connector 170 for transmitting data to and from the electronic board 190 to an external transmitting or receiving means. In one embodiment of the present invention, the electrical connector 170 may be used to program a chip that may be located in the electronic component area or areas of 146 and/or 147 of the electronic board 190 , for example with a computer. In another embodiment, the electrical connector 170 may be used for downloading video or still images that are captured by the camera that is located at the distal tip 120 of the probe piece means and stored in a memory chip that may be located in the electronic component area or areas of 146 and/or 147 of the electronic board 190. Additionally this memory chip may be removable from the present invention or reattached to the present invention. In another embodiment of the present invention the electronic connector 170 may be used to send video signal to an external monitor. In yet another embodiment, the electrical connector 170 may have an external device, such as a wireless adapter, should a wireless system not already be included within present invention, as it may be in one embodiment, attached to it to wirelessly send data from the present invention to an external receiving device, for example a monitor, or send and receive data wirelessly to and/or from, for example, a computer or other computing devices.

As mentioned previously, there is also attached to the electronic board 190 a switch 180 for turning on and off the present device. In some embodiments, the switch 180 would allow for power from the battery 195, shown in FIG 1B, to pass to the electronic board 190.

There is also attached to the electronic board 190, such as to electronic components located at either/or electronic component areas 146 and 147, a series of switches 145 for control of the present invention, as shown in FIG. 1I. In this embodiment there are three such switches 145 for controlling the present invention, but the number of switches 145, for example 1 to 10 switches, may be present on this device depending the number of controls required for different embodiments of the present invention. One example of what a switch 145 may control is image capture from the camera. Another example of what a switch may be used for is sending data, such as still images, from a memory source within this device, to an external source, for example a computer. Yet another example of what a switch may be used for is to control the illumination within the present invention. As previously mentioned, there is a plurality of means for the switches to control, and the number of controls on embodiments of this invention will be relative to such needs.

Additionally attached to the electronic board 190, such as to electronic components located at either/or electronic component areas 146 and 147, is a switch 140 for controlling the rotation of the motor which then controls the rotation of the catheter piece. In one embodiment, the switch 140 may be configured to have one of three positions whereby there is a neutral position in the middle, for example, and a position on either side on the neutral position for rotating the motor either clockwise or counter-clockwise as would be determined by the user's input.

Another attachment to the electronic board 190, and where desired to electronic components located at either/or electronic component areas 146 and 147, are a series of connectors 199. These connectors 199 may serve a variety of functions, including for the control of the motors 157 or 156, the camera 122, the lighting 126, and the monitor 130. In another embodiment, the connectors are linked to a sensor located at the distal tip 120 of the catheter.

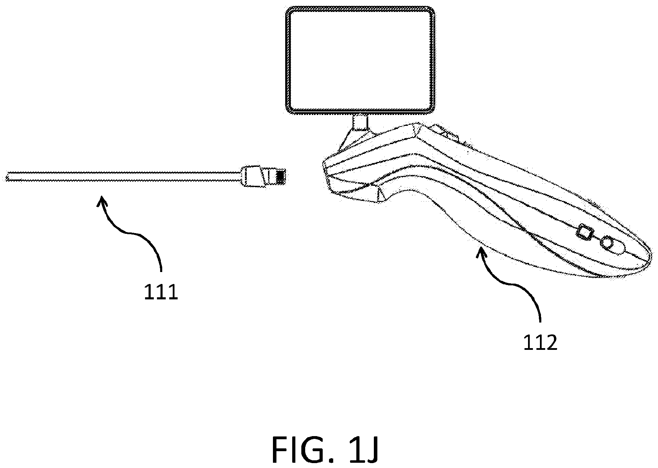

As shown if FIG. 1J, the portable diagnostic imaging system 100 has a connector to connect and detach the probe piece 111 of the device 100 from the hand piece 112 of the device 100. In one embodiment, the purpose of attaching and detaching the probe piece 111 of the device 100 from the hand piece 112 of the device 100 is to change the probe piece 111 from one embodiment of the probe piece 111 to another as would be the case where the two of more different probe pieces 111 have different functionality as required by the practitioner. In another embodiment of FIG. 1J, the purpose of detaching the probe piece 111 of the device 100 from the hand piece 112 is for the sterility requirements that the practitioner must follow, e.g., for a medical application. For example, should the practitioner require to use the device 100 with two of more patients, the practitioner would be required to dispose of the probe piece 111, and attach a new sterile probe piece 111 to hand piece 112.

In another embodiment of the current device 100, the monitor 113 may also be detachable from the hand piece 114 as shown in FIG. 1K. The functionality of detaching the monitor 113 from the hand piece 114 is to aid the practitioner with the viewing of the camera in a different location. In this case, the monitor 113 would be wirelessly connected to the hand piece 114 to allow video signals to be sent from the electronics within the hand piece 114 to the monitor 113.

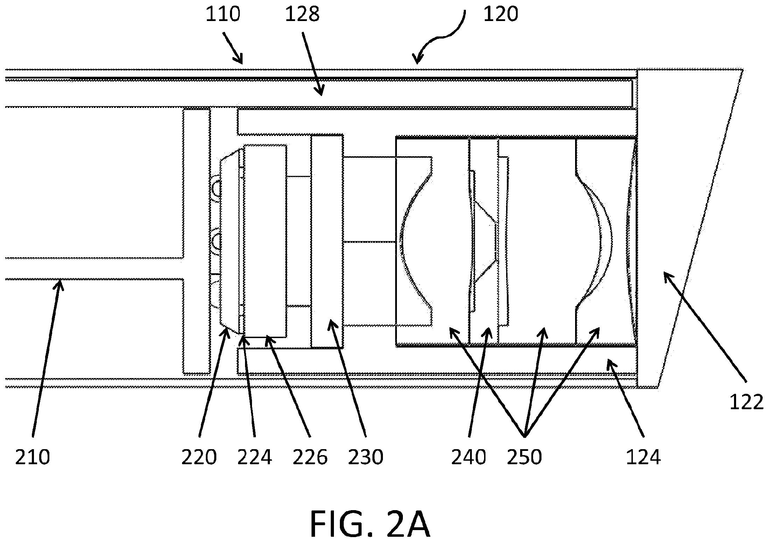

FIG. 2A shows a section view of the distal tip 120 of the probe piece 111. Shown in FIG. 2A are the necessary components that make up a camera and lighting module to produce an image that can be displayed on a monitor. The camera and lighting module as described allow viewing off-axis, and therefore make up an off-axis viewing module, as explained in greater detail below. A prism lens 122 covers the end of the elongated member 110 of the probe piece 111. The purpose of the prism lens 122 is to allow for imaging at angle to the axis of the probe piece, for example, 30 degrees. Proximal to the prism lens, in one embodiment, is shown a camera housing 124. Contained within this housing 124 is a series of lenses 250, an aperture 240, filters 230 and 226 and a CMOS imaging chip 220 that is attached to filter 226 by adhesive 224. In other embodiments of the camera, there may be more or less components as required to produce a different image. In addition, the chip 220 is mechanically and electrically attached to a circuit board 210 that transmits signals between the chip 220 and the electronics within the hand piece of the present invention. Also located within the distal tip 120 of the catheter piece is an integrated illuminator 128. In one embodiment, the integrated illuminator may be a fiberoptic bundle connected to an LED or other light source that is powered from the battery within the hand piece. In another embodiment, the integrated illuminator 128 may be a made from a light piping material such as a plastic or light transmitting hard resin or light transmitting liquid or air, all of which would be connected to an LED or other light source within the hand piece 114, as mentioned previously.

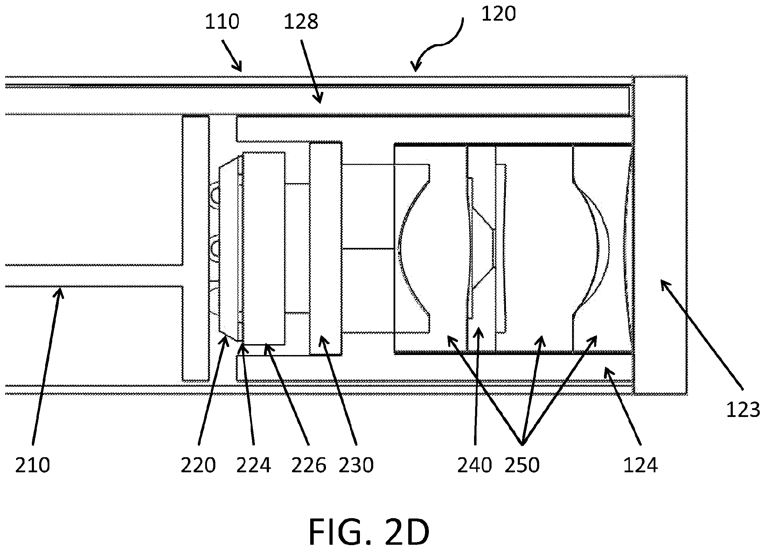

In another embodiment, of the components within the distal tip 120 as shown in FIG. 2D, a cover glass 123, is located in place of the prism lens 122 of FIG. 2A. In this case, a cover glass 123 allows the viewing of an image that is directly in from of the sensor chip 224. This configuration is an example of an "on-axis" imaging module.

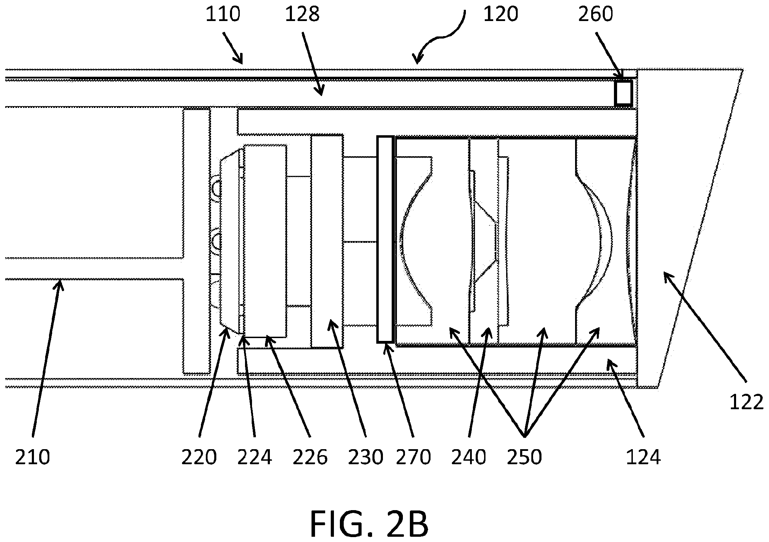

One challenge with an integrated illuminator 128 and a camera being mechanically located behind a prism 122 is that stray or unintended light from the integrated illuminator 128 or other source may interfere with the camera, thereby producing sub-optimum image. To address this issue, a visualization module may include a filtering system. FIG. 2B is one embodiment of a filtering system for controlling the incidence of light form the integrated illuminator 128 or other source of light, into the chip 220. Filter 260 is polarized opposite to filter 270 so that unintended light, particularly from the integrated illuminator 128 contained within the distal tip 120 of the catheter piece is less likely to enter the camera.

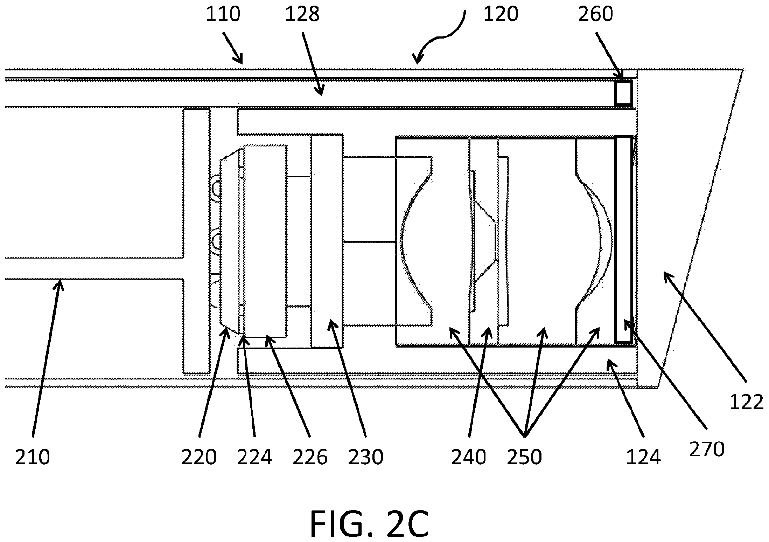

In another embodiment of the filtering means, as shown in FIG. 2C, the polarizing filter 270 is located distal to the lenses 250 contained within the camera housing 124, but proximal to the prism lens.

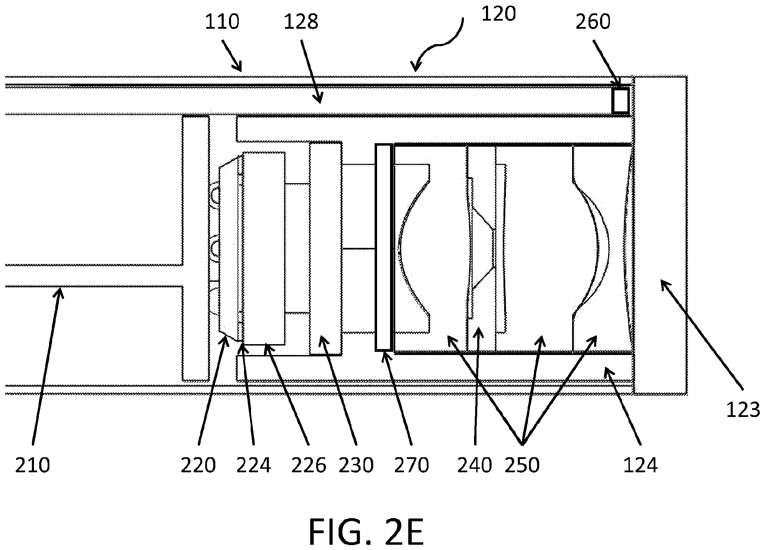

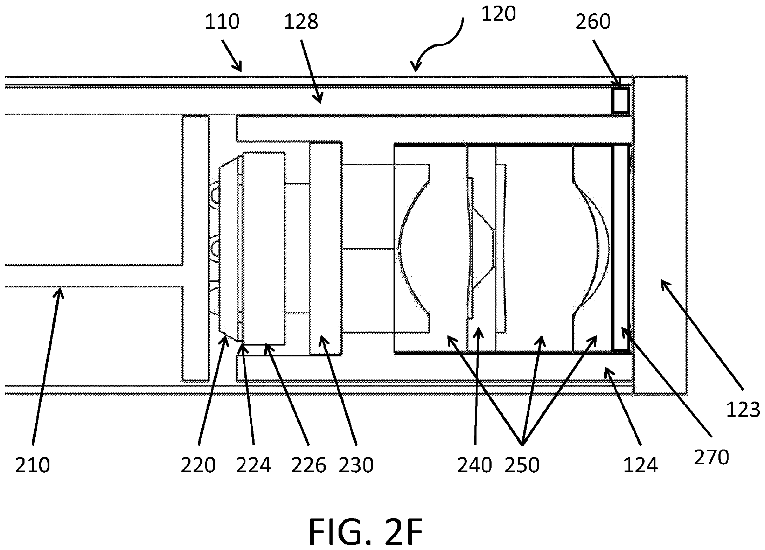

FIGS. 2E and 2F, are embodiments of a filtering system for controlling the incidence of light form the integrated illuminator 128 or other source of light into the sensor chip 220 as previously described and shown in FIGS. 2B and 2C, with the exception that the filters as shown in FIGS. 2E and 2F, are proximal to a cover glass 123 rather than a prism lens 122 as shown in FIGS. 2B and 2C.

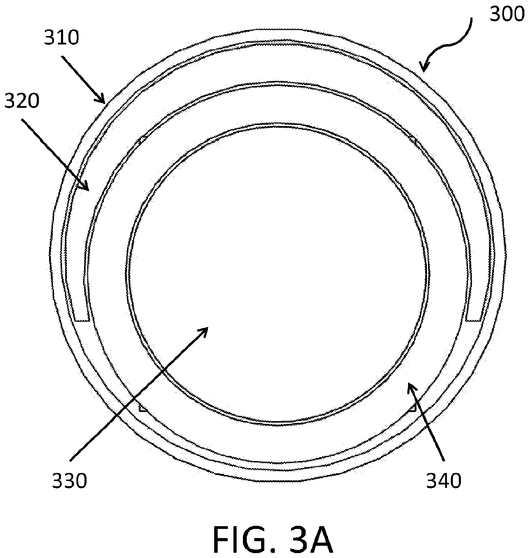

With reference now to FIGS. 3A-3D, there is shown an endways view of several embodiments for the mechanical arrangement of components located at the distal end 300 of a probe piece of device. As shown in FIG. 3A, an endways view of the probe-piece wall 310 has located eccentrically within its inner perimeter, a camera housing 340, camera lens and visualization sensor 330. In addition, an endways view of an integrated illuminator 320, such as the end of a fiber optic bundle, is located in the space between the camera housing 340 and inner perimeter of the probe piece wall 310. The integrated illuminator 320 has a crescent configuration so as to conform to the camera housing structure.

FIG. 3B illustrates the end of a probe piece that is analogous to that shown in FIG. 3A. In FIG. 3B, a non-visualization sensor (e.g., a pressure sensor) 390 is located on one side of the probe piece and a port 391 is located on the opposite side of the probe piece. Port 391 may be in operable connection to a lumen running at least part of the length of the probe piece, and may serve a variety of functions, including those described above, such as delivery of an active agent, etc.

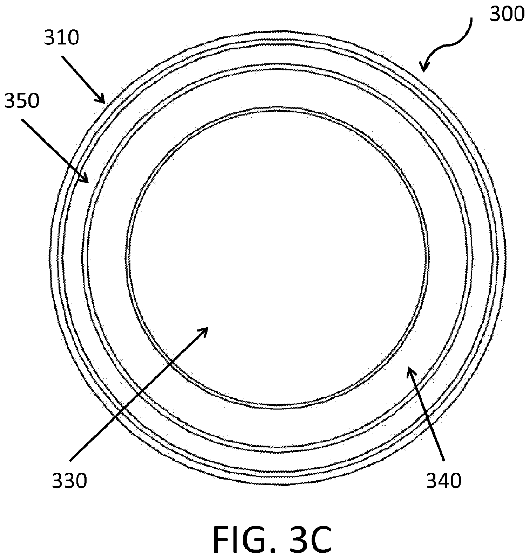

Another embodiment, for the mechanical arrangement of components located at the distal end 300 of the device, is shown in FIG. 3C. An endways view of the probe piece wall 310 has located concentrically within its inner perimeter, a camera housing 340 and camera lens and visualization sensor 330. In addition, an endways view of an integrated illuminator 350, such as the end of a fiber optic bundle, is located in the space between the camera housing 340 and inner perimeter of the probe piece wall 310.

FIG. 3D illustrates the end of a probe piece that is analogous to that shown in FIG. 3C. In FIG. 3D, a non-visualization sensor (e.g., a pressure sensor) 390 is located on one side of the probe piece and a port 391 is located on the opposite side of the probe piece. Port 391 may be in operable connection to a lumen running at least part of the length of the probe piece, and may serve a variety of functions, including those described above, such as delivery of an active agent, etc.

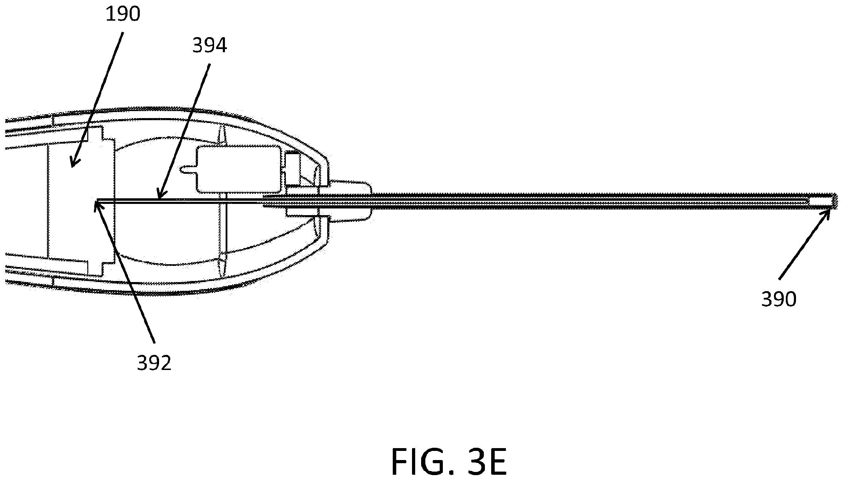

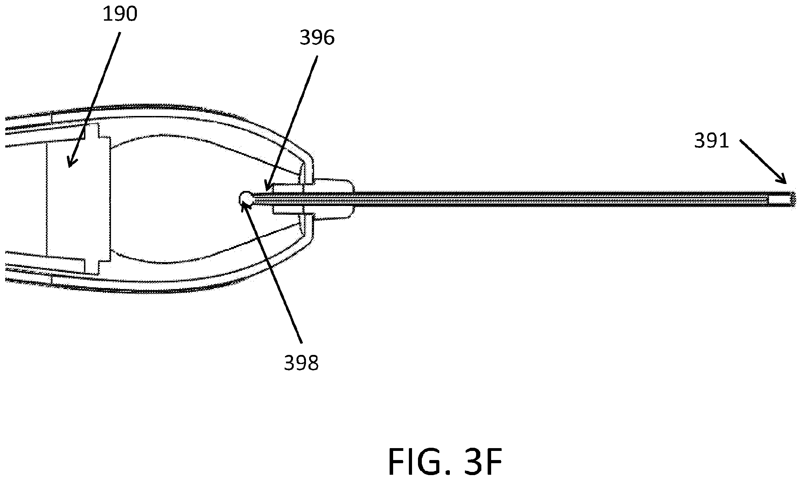

Data transfer from the sensor to a control module in the hand piece of the device may be accomplished using any convenient approach. In certain embodiments, transferring information from sensor 390 to the electronics within the hand piece is accomplished by a connection to the electronic board 190 at a point 392 via wires 394 that are passed through the probe piece from the sensor 390 into the hand piece, as shown in FIG. 3E. FIG. 3F illustrates one embodiment of a connection from a port 391, located at the distal end of the probe piece, to a port 398 in the hand piece via an open conduit 396, for example a tube, that passes between the ports, 391 and 398, and through the inside of the probe piece.

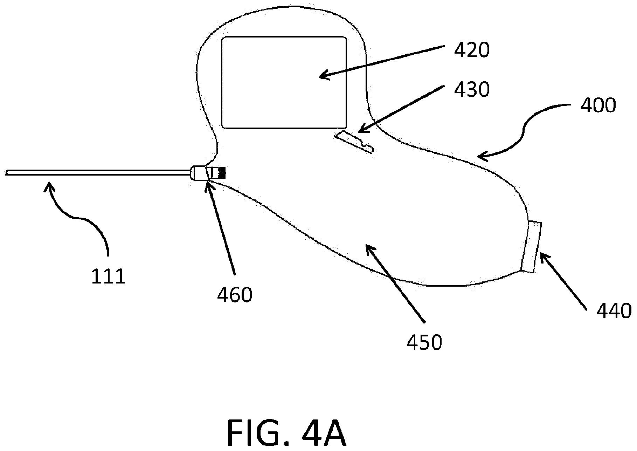

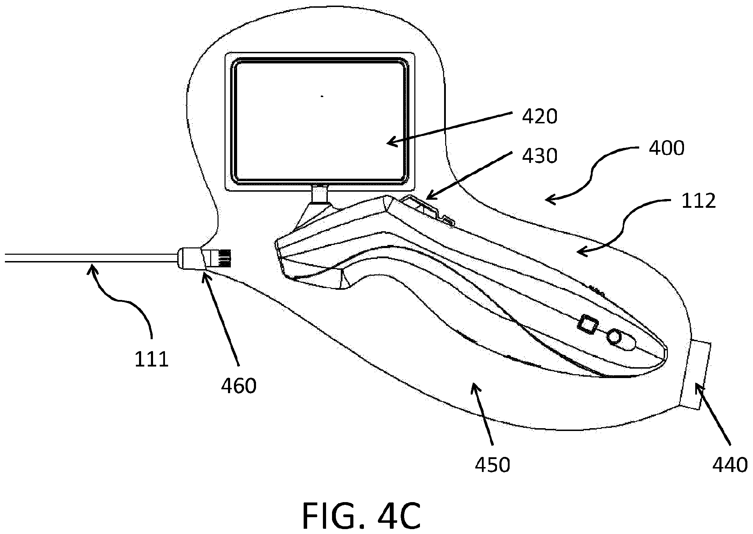

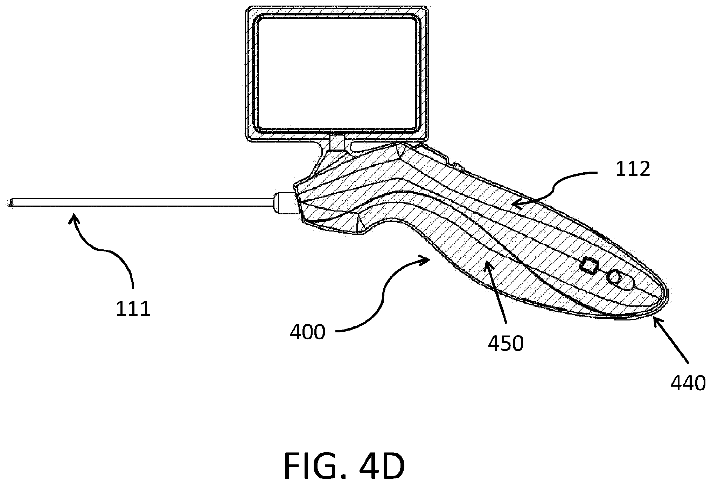

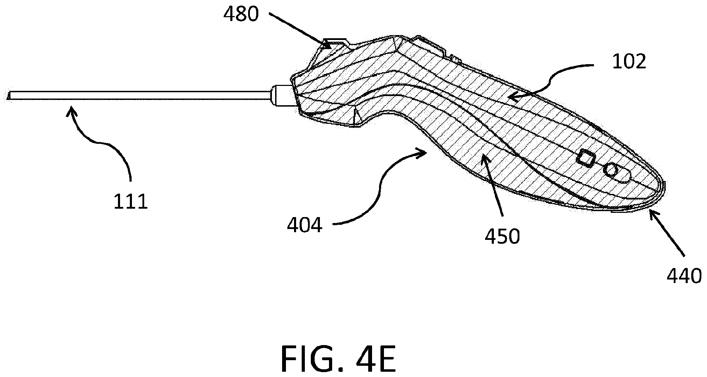

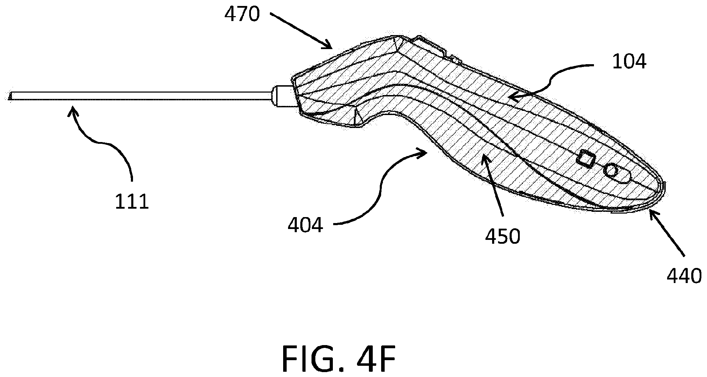

With reference now to FIGS. 4A-4F, there is shown several different embodiments configured to maintain sterility of the hand piece. As illustrated in FIGS. 4A to 4F, there is a sterile sheath (or bag), 400 or 404, that is sealably connected to the probe piece 111 at a location 460 circumferential to the probe piece 111. The sheath 400 or 404 includes a sheath piece 450. The sheath may also include one or more additional components, such as a clear monitor cover 420 and/or or a flexible boot 430. The sheath 400 or 404 is wrapped over an embodiment of the hand piece 112 (FIG. 4C and 4D), 102 (FIG. 4E), 104 (FIG. 4F), via an opening 440 in the hand piece portion of the sheath 450. Additionally, a seal is provided for sealing the sheath piece 450 at the opening 440 around an embodiment of the hand piece 112, 102, 104; for example, folding over the sheath piece 450 at the opening 440 and sealing it with tape or another method.

As mentioned above, and as shown in FIGS. 4A and 4C, an embodiment of the sheath 400 may have connected and sealed to it a rigid and clear monitor cover 420 and a flexible boot 430. The purpose of the monitor cover 420 is to allow for the functionality of the monitor means of the hand piece 112, while maintaining the sealability of the sheath 400. The monitor cover 420 may be comprised of a clear plastic, for example, that has the mechanical features to snap over the monitor means; the purpose of which is to allow for a clear view of the monitor for the practitioner of the present invention. The flexible boot 430 may be comprised of rubber, for example, that has the mechanical features to snap over the control elements, for example switches, of the hand piece 112, while maintaining the sealability of the sheath 400. With reference to FIG. 4D, the hand piece sheath portion 450 may then be sealed over the hand piece 112 at a location 440 as described previously.

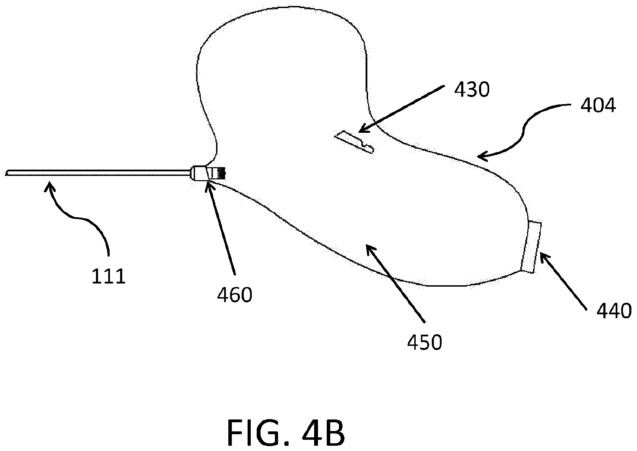

In another embodiment of the sheath 404, as shown in FIG. 4B, there is connected and sealed to it a flexible boot 430 as mentioned in the above embodiment, but without a monitor cover 420, FIG. 4A. The purpose of this embodiment of the sheath 404 is to be able to seal a hand piece 102, FIG. 4E, that has no monitor attached to it. In this case, there may be an attachment structure 480 located on the hand piece 102, where the monitor means may be attached and/or removed as required for use by the practitioner or the present invention.

In another embodiment of the sheath means 404, as shown in FIG. 4F, there is connected and sealed to the sheath piece 450 a flexible boot 430 as mentioned in the latter embodiment and without a monitor cover 420, FIG. 4A, for the purpose of sealing a hand piece 104 that has no feature 480, FIG. 4E, where the monitor may mount, located on the hand piece at a location 470, FIG. 4F.

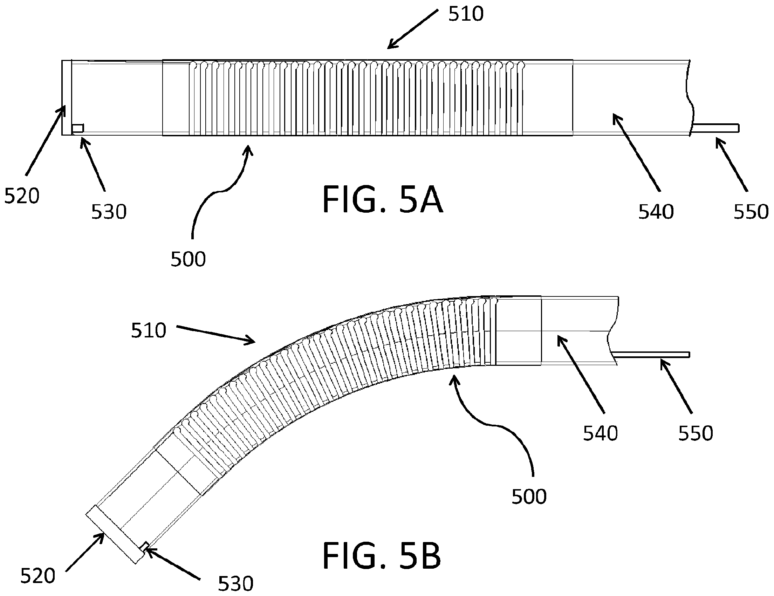

In one or more embodiments of the current invention it may be desirable to have the camera viewing in one or more directions, for example at an angle from the axis of the catheter piece, other than those directions that may be attained through the rotation of the catheter piece. The direction that the camera shall view may be controllable or fixed. With reference now to FIGS. 5A-5B, there is shown one embodiment for a flexible and controllable portion 500 of the probe piece. In this embodiment, a control cable 550, for example a twisted wire or rod, is connected at a distal location 530 to and within the tubular probe piece portion 540, and behind a distal lens 520. The control cable 550 joins to a control, for example a mechanical switch, within the hand piece, where it may be actuated, for example pulled toward the proximal end of the device. The actuation of the control cable, in this method, would cause the flexible portion 500 of the probe piece to bend as shown in FIG. 5B. The flexible portion 500 of the probe piece may then be returned to the position as shown in FIG. 5A, for example, by a spring means, or possibly by the actuation of the control cable 550 towards the distal end of the device.

The flexible portion 500 of the probe piece may be constructed in such a way as to allow for flexion of this portion of the probe piece, in one or more directions. The embodiment as shown in FIGS. 5A-5B shows one example of how to create the flexible portion 500 of the probe piece, by having a series of cut-outs covered with a hydrophobic tube 510. In this case the flexible portion 500 is configured to flex in one direction, that being shown in FIG. 5B. In addition, the purpose of the hydrophobic tubing surrounding the cut-outs 510 is to prevent material ingress into the probe piece, for example water, while allowing for the flexion of the flexible portion 500. Depending on the number and orientation of the cut-outs, this flexible portion 500 may be flexible in a plurality of directions and degrees, and may be controlled by a concomitant number of control cables connected to switches or other mechanical controls within the hand piece.

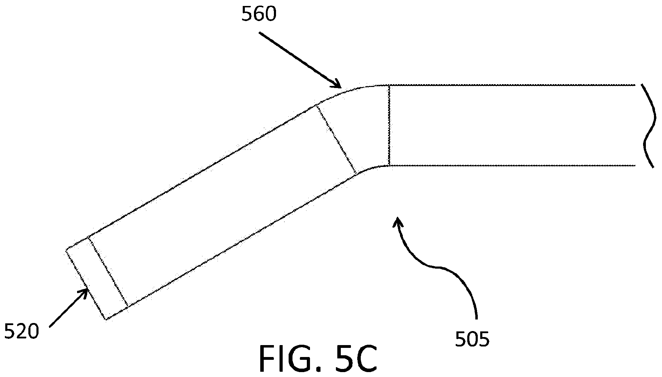

Another embodiment for the viewing of the camera at an angle, for example 30 degrees from the central axis of the catheter piece, is shown in FIG. 5C. In this case, there is an angle formed at a bend 560 in this portion of the catheter piece 505 which terminates at the proximal end of a lens 520 at the distal tip of the catheter piece. The bend 560 in this portion of the catheter piece 505 may be rigid, such as the case of a bent steel tube, or flexible, as would be the case, for example, of a formed flexible plastic tube. In the case where the formed bend 560 is flexible, there may be a spring inside, such as a NITINOL™ wire, that is configured to provide for the temporary bending of this portion 505 into a straight position, aligned with the central axis of the catheter piece, by the practitioner, and when released would bend back to the formed position.

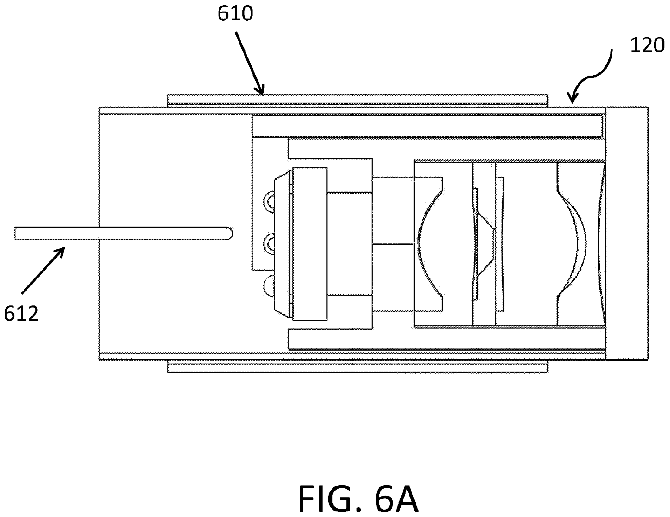

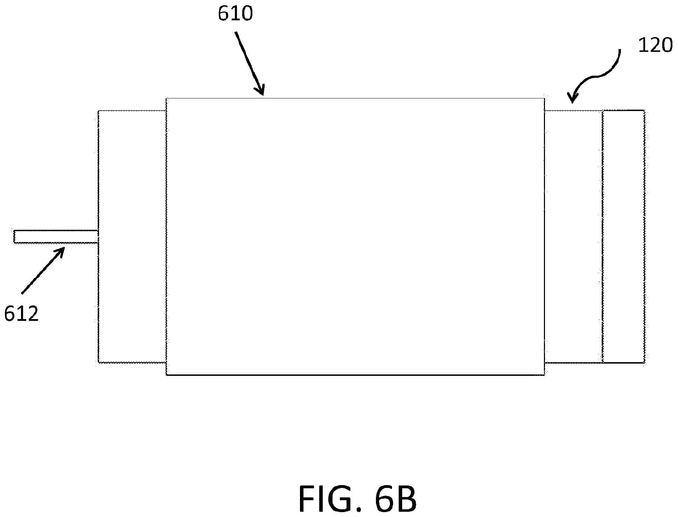

In cases where the practitioner of the present invention is required to diagnose, for example a tissue, it may be required of the practitioner to retrieve a portion of the material under diagnosis. With reference now to FIGS. 6A-6B, there is shown one embodiment of a controllable low-profile biopsy tool. FIG. 6A shows a section view of one embodiment of the distal tip 120 of the probe piece. FIG. 6B shows an external side view of one embodiment of the distal tip 120 of the probe piece. In this case, there is a low-profile biopsy tool that includes a cutting piece 610 and a control piece 612. Cutting piece 610 is concentrically disposed about the distal end of the probe piece 120, and configured to be moved relative to the distal end of the probe piece 120 in a manner sufficient to engage tissue. The control piece 612, for example a rod, may be attached to the cutting piece 610, and it may extend to the hand piece where is would be actuated by a mechanical means.

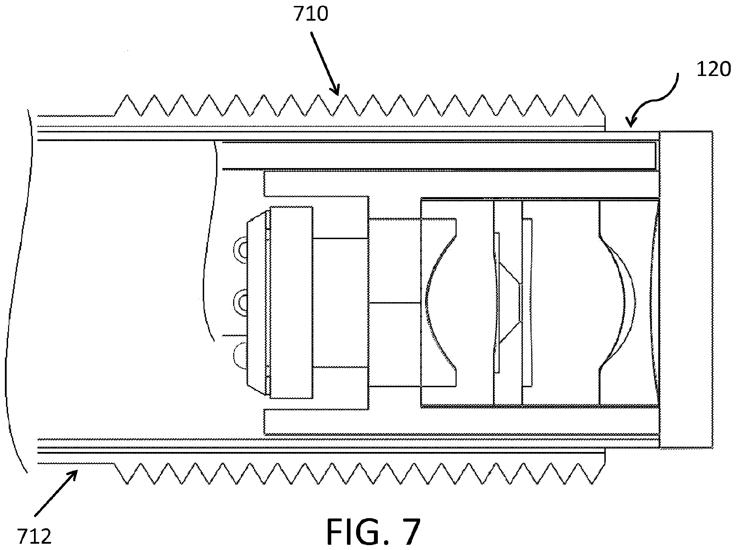

There may be cases where the practitioner of the present invention is required to scrape or cut material, for example a tissue. With reference now to FIG. 7, there is shown one embodiment of a cutting or scraping tool. This figure shows a section view of one embodiment of the distal tip 120 of the probe piece. In this case, there is a low-profile cutting or scraping tool that includes a cutting piece 710 and a control piece 712, and is concentrically disposed about the distal end of the probe piece 120. This tool may be configured to be moved relative to the distal end of the catheter piece 120 in a manner sufficient to engage material, for example tissue. In another embodiment, this tool may be configured to be rotated circumferentially to the distal end of the catheter piece 120 in a manner sufficient to engage material, for example tissue. In yet another embodiment, this tool may be fixed at the distal end of the catheter piece 120. The control piece 712, for example a tube or rod, may be attached to the cutting piece 710, and it may extend to the hand piece where is would be actuated by a mechanical means should that be necessary for the particular embodiment of the tool.

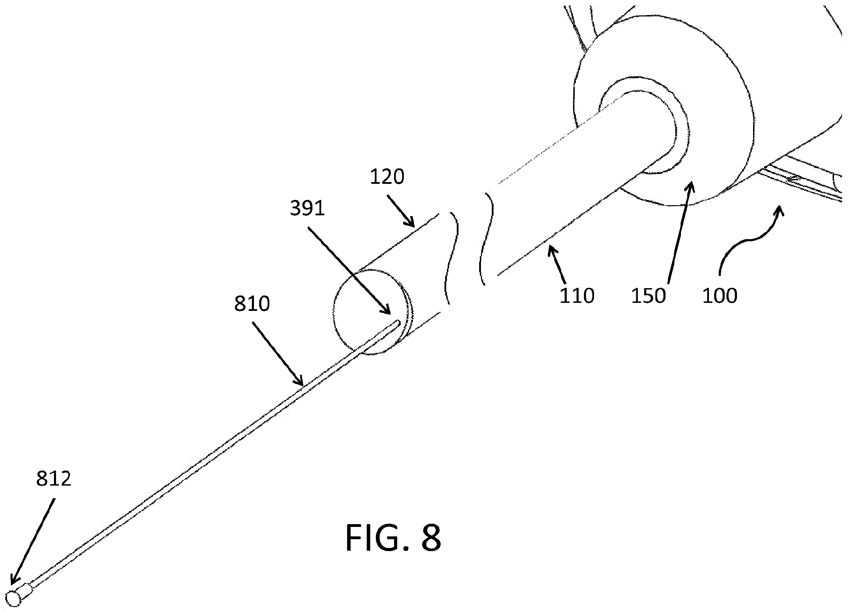

There may be cases where the practitioner of the present invention is required to deploy one or more sensors in or near or around a material, for example a tissue. Such may be the case in a diagnosis of a material, for example a tissue, where monitoring the material in question requires a continuous sensing and also requires the removal of the visualization means of the present invention from, for example a patient under diagnosis. With reference now to FIG. 8, there is shown one embodiment of a deployable sensor 812 incorporated into a device of the present invention 100 by a wired connection 810. Alternatively, a wireless communication module may be employed instead of wired connection 810. As illustrated, the wired connection passes through a port 391, as shown in FIGS. 3B and 3D where it then passes through the distal tip 120 of the probe piece and the elongated member 110 of the probe piece and the connector 150 of the probe piece. The wired connection 810 then connects to the electronics board within the hand piece where its output may be processed. This processed output may be displayed on a monitor and/or recorded to a memory chip on the electronics board, for example. The wired connection 810 may have sufficient slack, for example extra wire length, so as to allow the sensor to be located at some distance, for example 200 mm, from the visualization sensor. In one embodiment, the deployable sensor 812 may have mechanical features that aid in the deployment of the sensor, for example a hook or a spike or a barb.

As mentioned previously, there may be a wireless deployment of the sensor 812. In this case, the sensor 812 would wirelessly connect to the electronics board within the handle where its output would be processed. Any convenient wireless communication protocol may be employed. This processed output may be displayed on a monitoring means and/or recorded to a memory chip on the electronics board, for example.

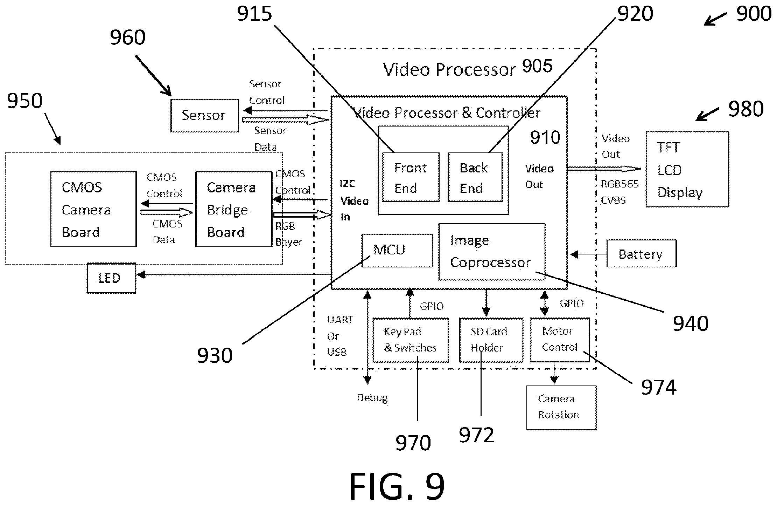

FIG. 9 illustrates a functional block diagram of a system 900 including a video processor module 905, according to one embodiment. Video processor module 905 includes a processor/controller module 910 which is in communication with sensor module 960, camera module 950, and display 980. Processor/controller module 910 comprises front end module 915, back end module 920, microcontroller 930, and image coprocessing module 940. Image coprocessing module 940 includes, for example, stereoscopic image module and performs the previously described functions and operations of the stereoscopic image module.

Camera module 950 may include a single visualization sensor, or two or more distinct visualization sensors which provide image data. Front end module 915 includes circuitry for receiving the image data from the camera module 950. The image data received from camera module 950 is processed by stereoscopic image module (i.e., by image coprocessing module 940) to provide stereoscopic image data. For example, as previously described, the image data from each distinct visualization sensor may be warped to correct image distortion, and fused to construct a single stereo image taking into account three-dimensional depth information. Back end module 920 includes circuitry for sending the stereoscopic image data to display 980. Display 980 displays a three-dimensional view of the image data for the user to see.

Video processor module 905 may be electrically coupled with camera module 950 via an I2C bus, for example, with camera module 950 configured as the slave and microcontroller 930 as the master. Microcontroller 930 may be configured to send camera control data to the camera module 950. The camera control data may comprise information requests (e.g., for information relating to testing/debugging, for calibration data, etc.) or provide commands for controlling the camera module 950 (e.g., controlling the two or more distinct visualization sensors, etc.).

Sensor module 960 may include one or more sensors and/or tools previously described. The one or more sensors and/or tools implemented may provide sensor data related to their specific function and application. The sensor data is received by processor/controller module 910 and may be used in a variety of ways depending on the specific function of the sensor(s) and/or tool(s) and their application. For instance, sensor data may be used by processor/controller module 910 to provide information to a user (e.g. parameter data, calibration data, measurement readings, warnings, etc., to be displayed on display 980 or to illuminate one or more LEDs), to account for feedback signals for more accurate control of a specific sensor(s) and/or tool(s), to store in memory, to further process into additional related information, etc. Microcontroller 930 may also control the sensor module 960 via the I2C bus or General Purpose Input/Output (GPIO) interface by sending sensor control data (e.g., to control and/or calibrate the specific sensors and/or tools implemented).

Processor/controller module 910 further comprises various modules for interfacing with external devices and peripherals. For example, as shown in FIG. 9, processor control module includes a key pad and switches circuitry 970 for receiving input signals from the user key pad and switches on the device; SD card holder circuitry 972 for sending/receiving data stored in memory devices, and motor control circuitry 974 for controlling the camera rotation. Microcontroller 930 may be configured with, for example, a GPIO to communicate with the various circuitry. Furthermore, the video processor module 905 may include a communication interface for implementing testing or debugging procedures--e.g., UART, USB, etc.

### METHODS

Aspects of the subject invention also include methods of imaging (and in some embodiments modifying) an internal target tissue of a subject. Accordingly, aspects of the invention further include methods of imaging an internal tissue site with tissue visualization devices of the invention. A variety of internal tissue sites can be imaged with devices of the invention. In certain embodiments, the methods are methods of imaging an intervertebral disc in a minimally invasive manner. For ease of description, the methods are now primarily described further in terms of imaging IVD target tissue sites. However, the invention is not so limited, as the devices may be used to image a variety of distinct target tissue sites.

With respect to imaging an intervertebral disc or portion thereof, e.g., exterior of the disc, nucleus pulposus, etc., embodiments of such methods include positioning a distal end of a minimally invasive intervertebral disc imaging device of the invention in viewing relationship to an intervertebral disc or portion of there, e.g., nucleus pulposus, internal site of nucleus pulposus, etc. By viewing relationship is meant that the distal end is positioned within 40 mm, such as within 10 mm, including within 5mm of the target tissue site of interest. Positioning the distal end in viewing device in relation to the desired target tissue may be accomplished using any convenient approach, including through use of an access device, such as a cannula or retractor tube, which may or may not be fitted with a trocar, as desired. Following positioning of the distal end of the imaging device in viewing relationship to the target tissue, the target tissue, e.g., intervertebral disc or portion thereof, is imaged through use of the illumination and visualization elements to obtain image data. Image data obtained according to the methods of the invention is output to a user in the form of an image, e.g., using a monitor or other convenient medium as a display means. In certain embodiments, the image is a still image, while in other embodiments the image may be a video.

The internal target tissue site may vary widely. Internal target tissue sites of interest include, but are not limited to, cardiac locations, vascular locations, orthopedic joints, central nervous system locations, etc. In certain cases, the internal target tissue site comprises spinal tissue.

In some instances, the methods may include obtaining a tissue biopsy with a low-profile biopsy tool. For example, the methods may include advancing an annular cutting member concentrically disposed about the distal end of the elongated member beyond the distal end of the elongated member in a manner sufficient to penetrate and engage target tissue. Following tissue engagement, the annular member may be retracted in the direction of the proximal end of the elongate member in a manner sufficient to secure an amount of tissue with the device which can then be removed from the body to obtain the tissue biopsy.

The subject methods are suitable for use with a variety of mammals. Mammals of interest include, but are not limited to: race animals, e.g. horses, dogs, etc., work animals, e.g. horses, oxen etc., and humans. In some embodiments, the mammals on which the subject methods are practiced are humans.

Aspects of the invention further include methods of assembling an internal tissue visualization device. In these embodiments, the methods include operatively coupling a proximal end of an elongated member to a hand-held control unit, e.g., as described above. Depending on the particular configuration, this step of operatively coupling may include a variety of different actions, such as snapping the elongated member into a receiving structure of the hand-held control unit, twist locking the elongated member into a receiving structure of the hand-held control unit, and the like. In some instances, methods of assembling may further include sealing the hand-held control unit inside of a removable sterile covering, where the sterile covering is attached to the proximal end of the elongated member and configured to seal the hand-held control unit from the environment, e.g., as described above. In such instances, the methods may further include sealing a proximal end of the sterile covering.

### UTILITY

The subject tissue visualization devices and methods find use in a variety of different applications where it is desirable to image an internal target tissue site of a subject while minimizing damage to the surrounding tissue. The subject devices and methods find use in many applications, such as but not limited to surgical procedures, where a variety of different types of tissues may be visualized, including but not limited to: soft tissue, cartilage, bone, ligament, etc. Specific procedures of interest include, but are not limited to, spinal fusion (such as Transforaminal Lumbar Interbody Fusion (TLIF)), total disc replacement (TDR), partial disc replacement (PDR), procedures in which all or part of the nucleus pulposus is removed from the intervertebral disc (IVD) space, arthroplasty, and the like. As such, methods of the invention also include treatment methods, e.g., where a disc is modified in some manner to treat an existing medical condition. Treatment methods of interest include, but are not limited to: annulotomy, nucleotomy, discectomy, annulus replacement, nucleus replacement, and decompression due to a bulging or extruded disc. Additional methods in which the imaging devices find use include those described in United States Published Application No. 20080255563.

### KITS

Also provided are kits for use in practicing the subject methods, where the kits may include one or more of the above devices, and/or components thereof, e.g., elongated members, hand-held control units, sterile coverings, etc., as described above. The kits may further include other components, e.g., guidewires, access devices, fluid sources, etc., which may find use in practicing the subject methods. Various components may be packaged as desired, e.g., together or separately.

In addition to above mentioned components, the subject kits may further include instructions for using the components of the kit to practice the subject methods. The instructions for practicing the subject methods are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

The following examples are offered by way of illustration and not by way of limitation.

### EXPERIMENTAL

A hand-held minimally dimensioned diagnostic device having integrated distal end visualization was constructed as follows. The device consisted of an outer SLA shell in the form of a hand-held unit housing batteries, a 3.5" monitor, a control board, and wires that connect to 2 LEDS and a visualization module at the distal tip of a steel 4mm hypodermic tube that was connected to the handle. The tubing was bent about an inch back from the distal tip to about 30 degrees. A manual wheel was provided on the hand-piece connected to the tube, and when actuated, rotated the tube 180 degrees in each direction. Considering a field of view for the camera of roughly 120 degrees (diagonal), the rotation of the tube allowed the camera to view at least a full hemisphere of space. The visualization module at the 4 mm outer diameter distal tip of the hypodermic tube included an Omnivision 6920 QVGA imaging chip (Santa Clara, CA), a series of lenses, an aperature, IR filter and a cover-glass within a small steel housing. In addition, LEDS were placed behind the flat cover-glass, but distal to the aperature. Thus due to the configuration of camera lens and lighting, there is little incidence of stray light affecting the image. In the constructed device, the signal from the powered camera goes through a series of electronic components where it is processed in a manner useful for the control board, and wires send the data to the control board where it is then displayed on the monitor. The monitor also rotates. QVGA resolution was observed for the image displayed on the 3.5 inch monitor.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims. It is also to be understood that the terminology used herein is for the purpose of describing particular embodiments only, and is not intended to be limiting, since the scope of the present invention will be limited only by the appended claims.

The scope of the present invention, therefore, is not intended to be limited to the exemplary embodiments shown and described herein. Rather, the scope of present invention is embodied by the appended claims.

## Claims

1. An internal tissue visualization device, the device comprising:
a hand-held control unit (114) comprising a hand piece;
an integrated visualization sensor configured to collect images of an internal tissue site and transmit the images to a monitor;
an elongated member (111) comprising a lumen wherein the lumen is configured to deliver a fluid to a tissue site and wherein the elongated member comprises a proximal end operatively coupled to the hand-held control unit and a minimally dimensioned distal end having integrated the visualization sensor; and
the distal end of the elongated member further comprising an integrated illuminator arranged concentrically around the visualization sensor and **characterized in that**: the hand-held control unit comprises a luer connector configured to receive the fluid; and
**in that** the integrated illuminator has a crescent configuration (320).

2. The device according to Claim 1, wherein the minimally dimensioned distal end has an outer diameter that is 3 mm or less.

3. The device according to Claims 1 or 2, wherein the integrated visualization sensor comprises a CMOS device.

4. The device according to any preceding claim, wherein the device is configured to reduce coupling of light directly from the integrated illuminator to the visualization sensor.

5. The device according to claim 4, wherein the device comprises a distal end polarized member, wherein the polarized member filters light reaching the visualization sensor.

6. The device according to any of the preceding claims, wherein the device comprises a removable sterile covering attached to the proximal end of the elongated member that is configured to seal the hand-held control unit from the environment.

7. The device according to any of the preceding claims, wherein the monitor further comprises an image processing module configured to receive image data of the tissue site from the visualization
sensor, and compare the received image data with a reference image of a tissue site comprising at least one of color descriptor data and anatomical descriptor data to make a determination as to whether an alert signal should be generated.

8. The device according to any of the preceding claims, wherein the elongated member comprises a distal end integrated non-visualization sensor, selected from the group consisting of: temperature sensors, pressure sensors, pH sensors, impedance sensors, conductivity sensors and elasticity sensors.

9. The device according to any of the preceding claims wherein the elongated member comprises a lumen that extends for at least a portion of the elongated member configured to deliver an agent to an internal tissue.

10. The device according to any of the preceding claims, wherein the elongated member is configured for distal end rotation.

## Patentansprüche

1. Visualisierungsvorrichtung für inneres Gewebe, wobei die Vorrichtung Folgendes umfasst:
eine tragbare Steuereinheit (114), die ein Handstück umfasst;
einen integrierten Visualisierungssensor, der dazu konfiguriert ist, Bilder einer inneren Gewebestelle zu sammeln und die Bilder an einen Monitor zu übertragen;
ein längliches Element (111), das ein Lumen umfasst, wobei das Lumen dazu konfiguriert ist, ein Fluid an eine Gewebestelle abzugeben, und wobei das längliche Element ein Proximalende, das mit der tragbaren Steuereinheit wirkverbunden ist, und ein möglichst gering bemessenes Distalende umfasst, in welches der Visualisierungssensor integriert ist; und
wobei das Distalende des länglichen Elements weiter einen integrierten Illuminator umfasst, der konzentrisch um den Visualisierungssensor angeordnet ist, und **dadurch gekennzeichnet, dass**: die tragbare Steuereinheit einen Luer-Verbinder umfasst, der dazu konfiguriert ist, das Fluid auszunehmen; und
dadurch, dass der integrierte Illuminator eine sichelförmige Konfiguration (320) aufweist.

2. Vorrichtung nach Anspruch 1, wobei das möglichst gering bemessene Distalende einen Außendurchmesser aufweist, der 3 mm oder weniger beträgt.

3. Vorrichtung nach Anspruch 1 oder 2, wobei der integrierte Visualisierungssensor eine CMOS-Vorrichtung umfasst.

4. Vorrichtung nach einem der vorstehenden Ansprüche, wobei die Vorrichtung dazu konfiguriert ist, ein Koppeln von Licht direkt von dem integrierten Illuminator mit dem Visualisierungssensor zu reduzieren.

5. Vorrichtung nach Anspruch 4, wobei die Vorrichtung ein polarisiertes Distalendeelement umfasst, wobei das polarisierte Element Licht filtert, welches den Visualisierungssensor erreicht.

6. Vorrichtung nach einem der vorstehenden Ansprüche, wobei die Vorrichtung eine abnehmbare sterile Abdeckung umfasst, die am Proximalende des länglichen Elements befestigt ist und die dazu konfiguriert ist, die tragbare Steuereinheit gegen die Umgebung abzudichten.

7. Vorrichtung nach einem der vorstehenden Ansprüche, wobei der Monitor weiter ein Bildverarbeitungsmodul umfasst, das dazu konfiguriert ist, Bilddaten der Gewebestelle von dem Visualisierungssensor zu empfangen und die empfangenen Bilddaten mit einem Referenzbild einer Gewebestelle zu vergleichen, das mindestens eines von Farbdeskriptordaten und Anatomiedeskriptordaten umfasst, um eine Bestimmung zu treffen, ob ein Alarmsignal generiert werden sollte.

8. Vorrichtung nach einem der vorstehenden Ansprüche, wobei das längliche Element einen in das Distalende integrierten Nicht-Visualisierungssensor umfasst, der ausgewählt ist aus der Gruppe bestehend aus: Temperatursensoren, Drucksensoren, pH-Sensoren, Impedanzsensoren, Konduktivitätssensoren und Elastizitätssensoren.

9. Vorrichtung nach einem der vorstehenden Ansprüche, wobei das längliche Element ein Lumen umfasst, das sich mindestens über einen Abschnitt des länglichen Elements erstreckt und dazu konfiguriert ist, ein Mittel an ein inneres Gewebe abzugeben.

10. Vorrichtung nach einem der vorstehenden Ansprüche, wobei das längliche Element für eine Drehung des Distalendes konfiguriert ist.

## Revendications

1. Dispositif de visualisation de tissu interne, le dispositif comprenant :
une unité de commande portative (114) comprenant une pièce à main ;
un capteur de visualisation intégré configuré pour collecter des images d'un site tissulaire interne et transmettre les images à un moniteur ;
un élément allongé (111) comprenant une lumière dans lequel la lumière est configurée pour délivrer un fluide à un site tissulaire et dans lequel l'élément allongé comprend une extrémité proximale fonctionnellement couplée à l'unité de commande portative et une extrémité distale aux dimensions minimales dans laquelle est intégré le capteur de visualisation ; et
l'extrémité distale de l'élément allongé comprenant en outre un illuminateur intégré agencé concentriquement autour du capteur de visualisation et **caractérisé en ce que** : l'unité de commande portative comprend un raccord luer configuré pour recevoir le fluide ; et
**en ce que** l'illuminateur intégré a une configuration de croissant (320).

2. Dispositif selon la revendication 1, dans lequel l'extrémité distale aux dimensions minimales a un diamètre extérieur qui est de 3 mm ou moins.

3. Dispositif selon les revendications 1 ou 2, dans lequel le capteur de visualisation intégré comprend un dispositif CMOS.

4. Dispositif selon l'une quelconque des revendications précédentes, dans lequel le dispositif est configuré pour réduire le couplage de lumière directement de l'illuminateur intégré au capteur de visualisation.

5. Dispositif selon la revendication 4, dans lequel le dispositif comprend un élément polarisé d'extrémité distale, dans lequel l'élément polarisé filtre la lumière atteignant le capteur de visualisation.

6. Dispositif selon l'une quelconque des revendications précédentes, dans lequel le dispositif comprend une couverture stérile amovible fixée à l'extrémité proximale de l'élément allongé qui est configurée pour sceller l'unité de commande portative de l'environnement.

7. Dispositif selon l'une quelconque des revendications précédentes, dans lequel le moniteur comprend en outre un module de traitement d'image configuré pour recevoir des données d'images du site tissulaire du capteur de visualisation, et comparer les données d'image reçues à une image de référence d'un site tissulaire comprenant au moins les unes de données de descripteur de couleur et de données de descripteur d'anatomie pour permettre une détermination relative à la nécessité ou non de générer un signal d'alerte.

8. Dispositif selon l'une quelconque des revendications précédentes, dans lequel l'élément allongé comprend un capteur de non visualisation intégré à l'extrémité distale, sélectionné dans le groupe consistant en : des capteurs de température, des capteurs de pression, des capteurs de pH, des capteurs d'impédance, des capteurs de conductivité et des capteurs d'élasticité.

9. Dispositif selon l'une quelconque des revendications précédentes, dans lequel l'élément allongé comprend une lumière qui s'étend sur au moins une partie de l'élément allongé configurée pour délivrer un agent à un tissu interne.

10. Dispositif selon l'une quelconque des revendications précédentes, dans lequel l'élément allongé est configuré pour une rotation de l'extrémité distale.
